# EUROPEAN PATENT SPECIFICATION

(11) **EP 1 778 224 B1**
(45) Date of publication and mention of the grant of the patent: **26.03.2014**
(21) Application number: 05790718.0
(22) Date of filing: 14.07.2005
(51) Int. Cl.: A61K 31/553

(54) **FLT3 INHIBITORS FOR IMMUNE SUPPRESSION**
FLT3-INHIBITOREN ZUR IMMUNSUPPRESSION
INHIBITEURS DE FLT3 A DES FINS D'IMMUNODEPRESSION

(30) Priority: 19.07.2004 US 589511 P
(43) Date of publication of application: 02.05.2007
(73) Proprietor: THE JOHNS-HOPKINS UNIVERSITY, Baltimore, MD 21218 (US)
(72) Inventor: SMALL, Donald, Baltimore, MD 21208 (US); WHARTENBY, Katherine, A., Baltimore, MD 21212 (US); PARDOLL, Drew, Brookeville, MD 20833 (US)
(74) Representative: Adam, Holger
(86) International application number: PCT/US2005/025318
(87) International publication number: WO 2006/020145

(56) References cited:
- WO-A1-93/07153
- WO-A1-03/037347
- WO-A1-2004/058749
- WO-A2-02/36587
- WO-A2-2004/037784
- US-A1- 2002 160 974
- US-A1- 2003 219 827
- DATABASE WPI Week 200341 Thomson Scientific, London, GB; AN 2003-432951 XP002598633 & JP 2002 275068 A 25 September 2002 (2002-09-25)
- GILLILAND D GARY ET AL: "Therapeutic efficacy of All-trans-retinoic acid (ATRA) combined with the FLT3 inhibitor MLN518 in a murine model of acute promyelocytic leukemia" BLOOD, AMERICAN SOCIETY OF HEMATOLOGY, US, vol. 102, no. 11, 16 November 2003 (2003-11-16), page 66A, XP009136247 ISSN: 0006-4971
- O'FARRELL ANNE-MARIE ET AL: "SU11248 is a novel FLT3 tyrosine kinase inhibitor with potent activity in vitro and in vivo." BLOOD, vol. 101, no. 9, May 2003 (2003-05), pages 3597-3605, XP002598634 ISSN: 0006-4971
- LEVIS M ET AL: "A FLT3-targeted tyrosine kinase inhibitor is cytotoxic to leukemia cells in vitro and in vivo" BLOOD 20020601 US LNKD- DOI:10.1182/BLOOD.V99.11.3885, vol. 99, no. 11, 1 June 2002 (2002-06-01), pages 3885-3891, XP002598635 ISSN: 0006-4971
- SPIEKERMANN K ET AL: "The protein tyrosine kinase inhibitor SU5614 inhibits FLT3 and induces growth arrest and apoptosis in AML-derived cell lines expressing a costitutively activated FLT3" BLOOD, AMERICAN SOCIETY OF HEMATOLOGY, US LNKD- DOI:10.1182/BLOOD-2002-04-1045, vol. 101, no. 4, 15 February 2003 (2003-02-15), pages 1494-1504, XP002490467 ISSN: 0006-4971
- KELLY LOUISE M ET AL: "CT53518, a novel selective FLT3 antagonist for the treatment of acute myelogenous leukemia (AML)." CANCER CELL JUN 2002 LNKD- PUBMED:12124172, vol. 1, no. 5, June 2002 (2002-06), pages 421-432, XP002598636 ISSN: 1535-6108
- GRISWOLD ET AL.: 'Effects of MLN518, a Dual FLT3 and KIT inhibitor, on Normal and Malignant Hematopoiesis' BLOOD vol. 104, no. 9, 01 November 2004, pages 2912 - 2918, XP003008810

## Description

### RELATED APPLICATIONS

This application claims priority from U.S. Provisional Application No. 60/589,511 on July 19, 2004.

### FIELD OF THE INVENTION

The invention relates to FLT3 kinase inhibitors for suppressing an immune response of a cell, and preventing, or treating immune related disorders. Therapies provided by the invention include administering an FMS-related tyrosine kinase 3 (FLT3) inhibitor to a subject in need thereof, such as a subject suffering from organ rejection after transplantation, bone marrow transplant rejection, graft-versus-host disease or lupus. Methods for screening therapeutic agents for treating immune disorders as disclosed herein include the use of a mouse having an elevated level of FLT3 receptor activity.

### BACKGROUND

Immune related disorders, notably organ rejection after transplantation, diabetes, graft-versus-host disease, Graves' disease, and lupus, remain debilitating diseases affecting millions of people world-wide.

Immune related disorders can cause potentially devastating and irreversible side effects for an individual and may occur, for example, as a result of a heightened immune system. Immune related disorders, such as, bone marrow transplant rejection, solid organ rejection after transplant, ankylosing spondylitis, arthritis, aplastic anemia, Behcet's disease, Graves' disease, hemolytic anemia, hyper IgE syndrome, idiopathic thrombocytopenia purpura (ITP), multiple sclerosis (MS), rheumatoid arthritis, Wegener's granulomatosis, type 1 diabetes mellitus, Myasthenia gravis, and psoriasis are caused by various over activity of the immune system. Current therapies which for the most part act on the T cell arm of the immune response often result in severe immunosuppression.

US 2002/0160974 discloses treatment of an autoimmune disease by administering an interferon antagonist in combination with a FLT3 ligand antagonist.

Accordingly, it would be desirable to have new therapies for treatment against such indications.

### SUMMARY OF THE INVENTION

We now provide FLT3 kinase inhibitors for suppressing the immune response of a cell, thereby treating immune related disorders. In particular, by inhibiting signaling through FMS-related tyrosine kinase 3 (FLT3) FLT3 and FLT3 related disorders may be treated, for example, by decreasing the number of dendritic cells (DC) developing *in vivo* and by decreasing their ability to activate T cells.

Disclosed are methods of suppressing an immune response of a cell comprising contacting the cell with at least one FLT3 inhibitor that reduces the activity of FLT3.

In certain forms the cell is a dendritic cell, an NK cell, T-cell, a B-cell, or a nervous system cell, for example, a neuron, glial cell, oligodendrocyte, Schwann cell, astrocyte, microglia. Examples of neurons include, for example, one or more of an afferent neuron, efferent neuron, interneuron, GABAergic neuron, cholinergic neuron, dopaminergic neuron, serotonergic neuron, neuroendocrine cell, postmitotic neurons, embryonic neurons, or ganglion cells in retina.

The kinase activity of FLT3 is reduced. In a related option the autophosphorylation activity of FLT3 is reduced.

According to one aspect, the FLT3 inhibitor is selected from one or more of a small molecule, an anti-sense oligo-nucleotide, an anti-FLT3 antibody, an antigen-binding fragment of an anti-FLT3 antibody, a polypeptide, a peptidomimetic, a nucleic acid encoding a peptide, an organic molecule and any combination thereof. In a related embodiment, the small molecule is selected from one or more of CEP701, AG 1296, AG 1295, CEP-5214, CEP-7055, PKC412, SU11248, SU5416, SU5614, MLN518, BAY43-9006, CHIR-258, amino-benzimidazole-quinolones, Ki23819, staurosporine.

In a further related embodiment, the small molecule is a compound according to formula 1: 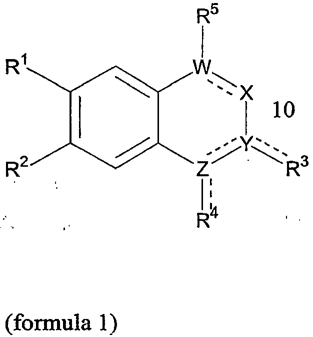 wherein:
W can be C, N, O;
X can be C, N, absent;
Y can be C;
Z can be C, N;
and R groups can be substituted variants of:
   R1 can be H, alkoxy, hydroxyl, heterocyclic alkyl/aryl;
   R2 can be H, F, alkoxy, heterocyclic alkyl/aryl;
   R3 can be H, O, aryl, heteroaryl, C(O)heteroaryl;
   R4 can be H, alkyl, aryl, heteroaryl, CH-heteoaryl, absent;
   R5 can be H, aryl, heteroalkyl/aryl, absent; and
   the dashed lines between WX, WY, YZ, YR3, and ZR4 indicating an optionally double bond, wherein the aryl, heteroaryl, heterocyclic alkyl/aryl are optionally substituted.

In yet another related embodiment, the small molecule is selected from: 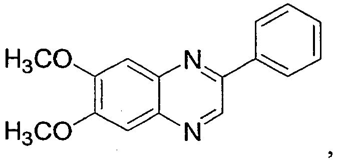 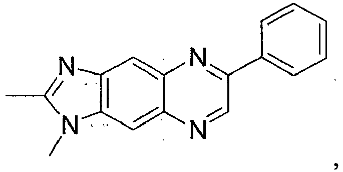 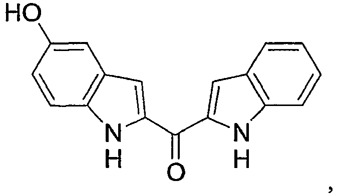 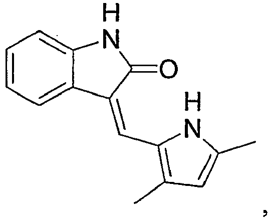 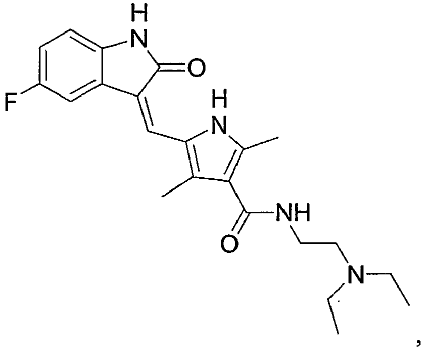 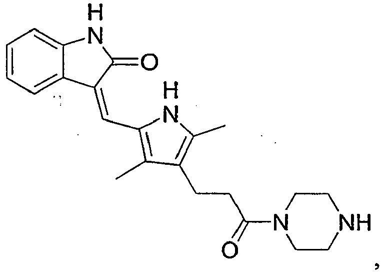 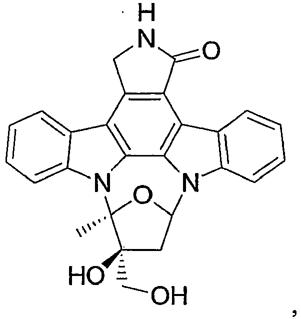 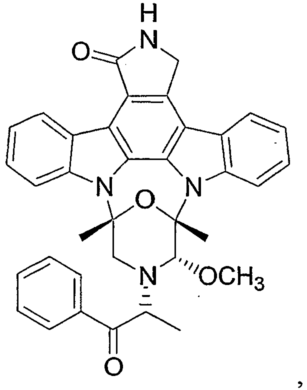 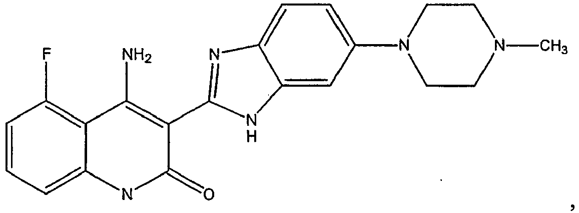 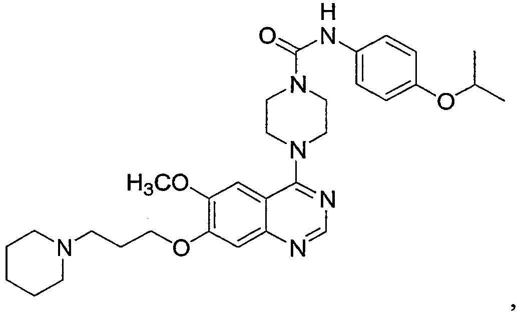 or

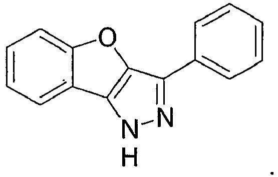

The antibody may be selected from one or more of IMC-EB10 and IMC-NC7. In a related embodiment, the antibody may be a monoclonal antibody, a chimeric antibody, a single chain antibody, an anti-idiotypic antibody, a humanized antibody, a fully human antibody, a primatized antibody and any combination thereof. In one embodiment, the antibody specifically recognizes a phosphorylated form of FLT3. In another embodiment, the antibody specifically recognizes an unphosphorylated form of FLT3.

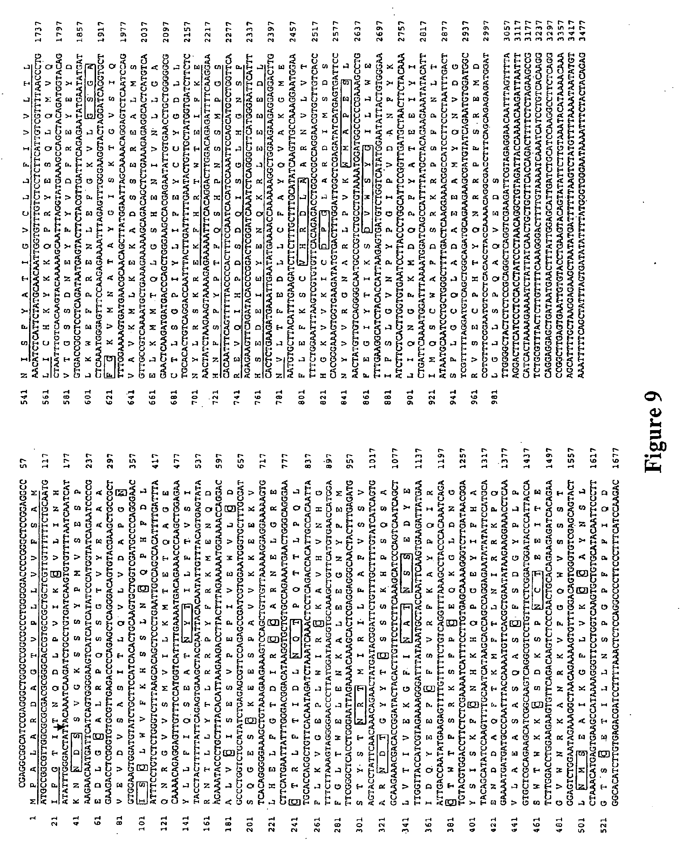

In a related embodiment, the anti-sense oligonucleotide is selected from one or more of the nucleotide sequence of Figure 9 or a biologically active fragment thereof.

In another embodiment, the FLT3 inhibitor reduces FLT3 dependent signal transduction.

In one embodiment, the FLT3 inhibitor downregulates the immunostimulatory capacity of the cell.

Certain methods may further comprise administering a T-cell inhibitor with the FLT3 inhibitor.

Methods of treating a FLT3 disorder or an FLT3 related disorder in a subject are provided. The methods comprise administering to the subject a therapeutically effective amount of an FLT3 inhibitor to reduce the activity of FLT3.

The treatment methods may further comprise identifying the subject as needing treatment for an FLT3 disorder or an FLT3 related disorder and/or whereby the subject is thereby treated for the FLT3 disorder or an FLT3 related disorder.

In one option the FLT3 disorder or related disorder is an immune related disorder.

In a related option, the immune related disorder is one or more of organ rejection, bone marrow transplant rejection, non-myeloablative bone marrow transplant rejection, ankylosing spondylitis, arthritis, aplastic anemia, Behcet's disease, type 1 diabetes mellitus, graft-versus-host disease, Graves' disease, autoimmune hemolytic anemia, Wegener's granulomatosis, hyper IgE syndrome, idiopathic thrombocytopenia purpura, rheumatoid arthritis, Crohn's disease, multiple sclerosis, Myasthenia gravis, psoriasis, and lupus, among other autoimmune diseases. It might also be used to enhance bone marrow engraftment after non-myeloablative conditioning regimens, and combinations thereof.

In one option, the FLT3 disorder or an FLT3 related disorder is a neurological disorder.

In a related option, the neurological disorder is one or more of a neurodegenerative disease, for example a disease caused by axonal degeneration. Neurodegenerative diseases include, for example, multiple sclerosis; demyelinating disorders, such as multiple sclerosis, acute transverse myelitis; and Creutzfeldt-Jakob disease; or Subacute sclerosing panencephalitis.

In one option, the subject is a mammal.

In another option, the mammal is a human, a primate, a rat, a dog, a cat or a mouse.

In one option, the FLT3 inhibitor is selected from one or more of a small molecule, an anti-sense oligo-nucleotide, an anti-FLT3 antibody, an antigen-binding fragment of an anti-FLT3 antibody, a polypeptide, a peptidomimetic, a nucleic acid encoding a peptide, an organic molecule and any combination thereof.

In one option, the anti-sense oligonucleotide is selected from one or more of the nucleotide sequence of Figure 9 or biologically active fragments thereof.

In another option, the FLT3 inhibitor reduces the kinase activity of FLT3 in the subject.

In one option, the FLT3 inhibitor reduces the autophosphorylation activity of FLT3 in the subject.

In one option, the FLT3 inhibitor reduces FLT3 dependent signal transduction in the subject.

In another option, the FLT3 inhibitor down-regulates the immunostimulatory capacity of the subject.

In one embodiment, further comprising administering a T-cell inhibitor with the FLT3 inhibitor is provided.

The invention provides methods of treating an FLT3 disorder or an FLT3 related disorder in a subject comprising administering to the subject a therapeutically effective amount of an FLT3 inhibitor to reduce FLT3 dependent signal transduction.

Also provided herein are transgenic non-human animals having a constitutively activated FLT3 receptor. In one embodiment, the transgenic animal is a mouse.

In another option, the transgenic animal is used to test therapeutic agents and/or as a model for immune related disorders.

Also provided are methods for screening a therapeutic agent for treating an immune related disorder comprising administering the agent to a mouse having an elevated level of FLT3 receptor activity, and measuring a change in the immune response, wherein a decrease in the immune response indicates that the agent may be useful in treating immune related disorders.

In one form, the change in the immune response is a decreased number of DC cells. In a related form, the change in the immune response is a decreased number ofNK cells. In another related form, the change in the immune response is measured by determining the level of DC costimulatory protein, wherein an increase in the level of DC costimulatory protein indicates that the agent may be useful in treating immune related disorders.

In one form, the DC costimulatory protein is B7-DC. In one form, the change in the immune response is measured by a T cell proliferation assay, wherein a decrease T cell proliferation indicates that the agent may be useful in treating immune related disorders.

Provided herein are methods for screening a therapeutic agent for treating an immune related disorder comprising administering a therapeutic agent to eukaryotic cells, and measuring a change in the immune response in the cell, wherein a decrease in the immune response of the cell indicates that the agent may be useful in treating immune related disorders.

In one form, the cell is selected from one or more of: dendritic cells, NK cells, spleen cells, T cells and mixtures thereof. In another form, the decrease in the immune response of the cell is measured by determining the level of DC costimulatory protein, wherein an increase in the level of DC costimulatory protein indicates that the agent may be useful in treating immune related disorders.

In another form, the DC costimulatory protein is B7-DC.

In another form, the change in the immune response is measured by determining T cell proliferation, wherein a decreased T cell proliferation indicates that the agent may be useful in treating immune related disorders.

Methods are provided for screening a therapeutic agent for treating a neurological disorder comprising administering the agent to a mouse that has been immunized with MOG peptide, and visualizing the neurons, wherein a modulation in the axons indicates that the agent may be useful in treating neurological disorders.

In one form, the change in the axons is a decrease in the number of degenerating axons.

In another form, the visualizing is by microscopic examination..

Provided herein are methods for screening a therapeutic agent for treating a neurological disorder comprising administering a therapeutic agent to neuronal cells, and measuring a change in the cells, wherein a decrease in the number of degenerating neurons indicates that the agent may be useful in treating an neuronal disorders.

Treatment methods include treating immune related disorders, including administering to a subject in need thereof a therapeutically effective amount of FLT3 inhibitor to reduce FLT3 activity. Treatment methods include administering to a subject in need thereof a therapeutically effective amount of an FLT3 inhibitor to reduce FLT3 dependent signal transduction. Treatment methods also include the breaking of tolerance in the setting of tumor vaccines.

Treatment methods include administration to a subject in need of such treatment a therapeutically effective amount of one or more therapeutic agents that can inhibit FLT3 signaling to an animal, including a mammal, particularly a human. Preferably, a subject is identified and/or selected that is susceptible to or suffering from an immune related disorder and then one or more FLT3 inhibitor compounds are administered to the identified and/or selected subjects.

Methods of screening therapeutic agents for treating immune related disorders include administering an agent to a mammas such as a mouse or other subject, having elevated levels of FLT3 activity and measuring the immune response. Methods of screening therapeutic agents for treating immune related disorders include administering an agent to a cell and measuring the immune response.

Other aspects of the invention are disclosed infra.

### BRIEF DESCRIPTION OF THE DRAWINGS

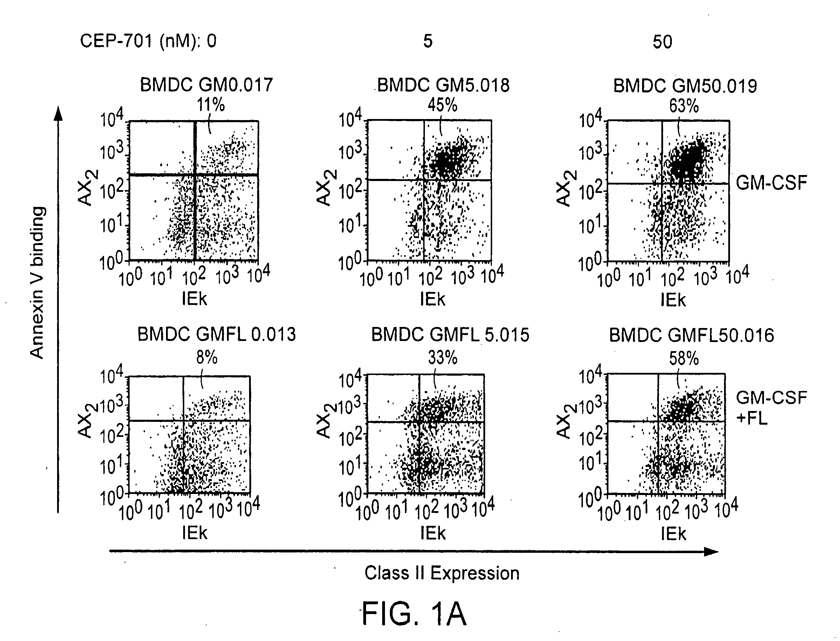

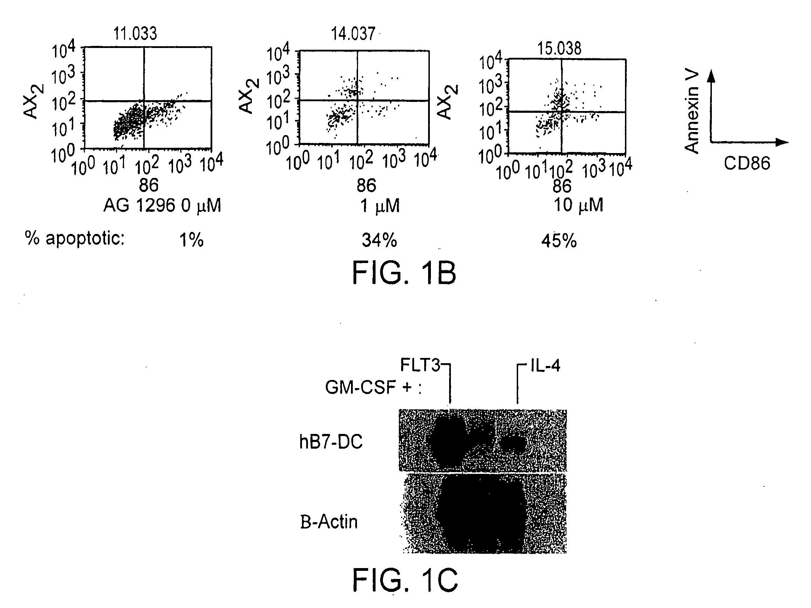

Figure 1a depicts treatment of DCs with the FLT3 inhibitor CEP-701. The results of DCs being incubated in a dose-response analysis to CEP 701 at 0, 5, or 50 nM concentrations is shown. The upper three graphs show cells stimulated with GM-CSF and the lower three show cells stimulated with GM-CSF and FLT3 ligand (FL). FLT3 inhibition results in the induction of apoptosis in a significant fraction of the DCs. Figure 1b depicts the results of treating DCs with the FLT3 inhibitor AG 1296 at 0, 1, and 10 µM and analyzing for apotosis. FLT3 inhibition results in the induction of apoptosis in a significant fraction of the DCs. Figure 1c depicts a Northern blot analysis of human bone marrow cells treated with FL and IL-4 or GM-CSF. Northern blotting was done for hB7-DC and beta-actin. This demonstrates that the addition of FL greatly up-regulates B7-DC expression (GM-CSF alone showed no expression; data not shown).

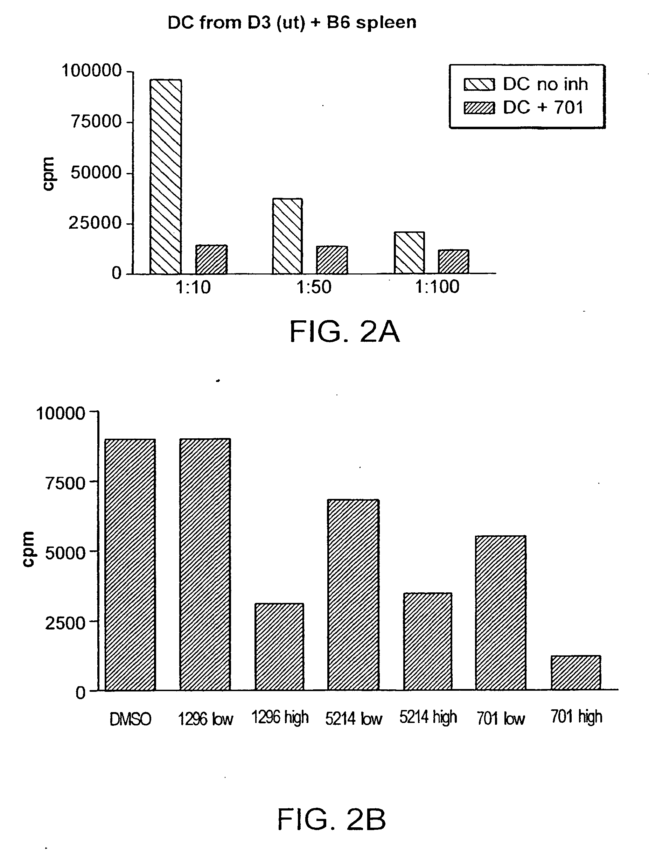

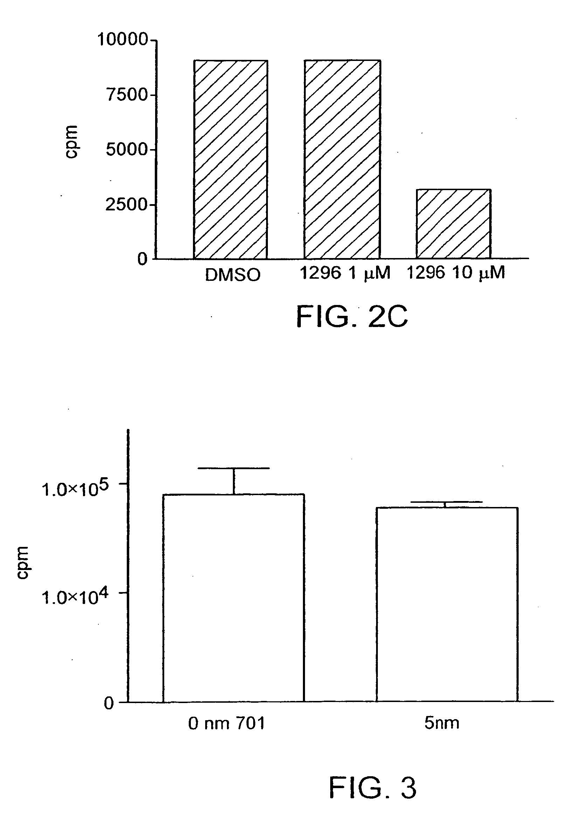

Figures 2a and 2b depict the results of FLT3 inhibition on DC and BALB/c splenocytes based on the proliferation of T cells. DCs were treated with CEP701 (or not as control) then plated with C57BL/6 splenocytes at the ratios shown. After 3 days, ³H was added, and the proliferation determined. CEP701 has a robust effect on decreasing proliferation of T cells. In figure 2b, BALB/c splenocytes were substituted for DCs, to test the inhibition of CEP701 on a standard mixed lymphocyte reaction (MLR). As the figure demonstrates, this response was also inhibited in the presence of CEP701. Figure 2c depicts the result of inhibition of BALB/c splenocytes by the FLT3 inhibitor AG1296.
Figure 3 graphically depicts the direct treatment of T cells with the FLT3 inhibitor CEP-701 after stimulation with anti-CD28 antibody. As shown, this treatment does not inhibit their proliferation.
Figures 4a and 4b graphically depict the outcome on immune cell populations of treatment of mice with the FLT3 inhibitor CEP701, which decreases CD11c and NK cell populations but not CD3 or B220 cell populations.
Figure 5 graphically depicts the treatment of mice with the FLT3 inhibitor CEP 701, which decreases an in vivo autoreactive immune response, demonstrating that inhibition of DCs is sufficient to downregulate an immune response.
Figure 6 depicts the treatment of DCs with AG1296 at either 0, 1, or 10 µM and then analyzed for the expression of co-stimulatory molecules B7.2 (CD 86) and B7-DC. As shown, both co-stimulatory molecules were down regulated in the presence of AG1296.
Figure 7 graphically depicts that DC stimulation of T cells is inhibited by AG1296.
Figure 8 graphically depicts that treatment with FLT3 inhibitors ameliorates established experimental autoimmune encephalitomyelitis (EAE).
Figure 9 is the complete and predicted amino acid sequence of STK-1 cDNA. Amino acids are numbered on the left of each column and nucleotides are numbered on the right. Underlined is the predicted signal peptide (aa 1-23) followed by the probable cleavage site marked with an arrow.
Figure 10 shows the number of degenerating axonal fibers in the defined field area for the sections of CEP-701 treated mice compared to vehicle control mice at 210 days post disease induction.

### DETAILED DESCRIPTION OF THE INVENTION

As stated above, and demonstrated in the examples which follow, it has now been found that inhibiting FLT3 can be effective to treat immune related disorders, including organ rejection, bone marrow transplant rejection, non-myeloablative bone marrow transplant rejection, acquired immune deficiency syndrome, ankylosing spondylitis, arthritis, aplastic anemia, Behcet's disease, type 1 diabetes mellitus, graft-versus-host disease, Graves' disease, hemolytic anemia, Wegener's granulomatosis, hypogammaglobulinemia, hyper IgE syndrome, idiopathic thrombocytopenia purpura, rheumatoid arthritis, Crohn's disease, multiple sclerosis, Myasthenia gravis, psoriasis, lupus and any combination thereof.

FLT3 is a receptor tyrosine kinase preferentially expressed in hematopoietic progenitor cells. (Small et al., Blood 15(4): 1110-9 (1993)). Signaling through the FLT3 receptor plays an important role in dendritic cells (DC) as evidenced by increasing numbers of DCs upon stimulation with its ligand FLT3 Ligand (FL). Signaling through FLT3 is known to affect hematopoietic stem and progenitor cells. FLT3 inhibitors can suppress downstream signaling through FLT3, which appears to be important for the development of T cells, NK cells and B cells. It has been surprisingly found that inhibiting FLT3 signaling leads to a decrease in the number of DCs and interferes with DCs ability to present antigen to activate T cells. FLT3 requires its tyrosine kinase domain to signal, thus, one method to inhibit FLT3 signaling is to inhibit this enzymatic activity. FL stimulation of FLT3 also greatly stimulates the development of NK cells and B lymphocytes, which are also important in the immune system and so the inhibition of FLT3 signaling would also have the effect of suppressing these cells as well.

FLT3 is expressed in DC progenitors, and FL/FLT3 signaling results in large increases in the numbers of DCs in bone marrow (BM), spleen, lymph nodes, GI tract, liver, lung, peritoneal caviey and skin. For example, see S.D. Lyman, Curr Opin Hematol 5, 192-6, (1998); H.J. McKenna, K.L., et al., Blood 95, 3489-97, (2000); E. Maraskovsky, et al., J Exp Med 184, 1953-62, (1996), FL-stimulated DCs are functional in mice and humans; M.R. Shurin, et al., Cell Immunol 179, 174-84, (1997); and E. Maraskovsky, et al., Blood 96, 878-84, (2000), GM-CSF also stimulates DC production. While either GM-CSF or FL dramatically increases DCs in vivo, the phenotype of the DCs generated appears to be somewhat different, suggesting that these cytokines may activate different pathways in DCs. For example, see E. Daro, et al., J Immunol 165, 49-58, (2000); K. Brasel, et al., Blood 96, 3029-39, (2000); P. Bjorck, Blood 98, 3520-6, (2001); and P.J. O'Connell, et al., J Immunol 165, 795-803, (2000). In particular, FL appears to expand both CD11b+/CD11c+ (myeloid) and CD11b-/Cd11c+ (lymphoid) DC subsets, whereas GM-CSF expands mainly CD11b-/CD11c+DCs.

Upon ligand binding in normal HSCs, FLT3 homodimerizes and its kinase domain is activated. FLT3 kinase activation has several consequences. FLT3 directly phosphorylates a number of substrate proteins on tyrosine residues, which in turn activates these proteins. In addition, FLT3 phosphorylates itself on several tyrosine residues, and these residues are then bound by a number of adapter proteins containing SH2 domains. Proteins that have been shown to be involved in FLT3 signaling include the p85 subunit of PI-3 kinase, PLCy, VAV, CBL, CRKL, Gab1 and 2, SHP2, SHIP, SHC, and STAT5. The end result is the transduction of signals that act on the nucleus to alter the genetic program of the cell. Some of these signals stimulate proliferation, whereas others appear to protect the cell from apoptosis or drive differentiation.

We have particularly found that the small molecule inhibitors of FLT3, for example, CEP701, AG 1296, AG 1295, and CEP-5214 can suppress the immune response through FLT3 signaling. We also have found that anti-FLT3 antibodies inhibit FLT3 signaling by inhibiting receptor activation, and antisense FLT3 molecules also prevent FLT3 expression, and thus inhibit an immune response through FLT3.

A method of suppressing an immune response of a cell comprises providing a population of eukaryotic cells, and contacting the cells with at least one FLT3 inhibitor that reduces the activity of FLT3. As used herein, the activity of FLT3 may refer to its kinase activity, its ability to transmit downstream signals, its autophosphorylation activity, and the like.

The term "administration" or "administering" includes routes of introducing the compound of the invention(s) to a subject to perform their intended function. Examples of routes of administration that may be used include injection (subcutaneous, intravenous, parenterally, intraperitoneally, intrathecal), oral, inhalation, rectal and transdermal. The pharmaceutical preparations may be given by forms suitable for each administration route. For example, these preparations are administered in tablets or capsule form, by injection, inhalation, eye lotion, ointment, suppository, etc. administration by injection, infusion or inhalation; topical by lotion or ointment; and rectal by suppositories. Oral administration is preferred. The injection can be bolus or can be continuous infusion. Depending on the route of administration, the compound of the invention can be coated with or disposed in a selected material to protect it from natural conditions which may detrimentally effect its ability to perform its intended function. The compound of the invention can be administered alone, or in conjunction with either another agent as described above or with a pharmaceutically-acceptable carrier, or both. The compound of the invention can be administered prior to the administration of the other agent, simultaneously with the agent, or after the administration of the agent. Furthermore, the compound of the invention can also be administered in a proform which is converted into its active metabolite, or more active metabolite in vivo.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. The term alkyl further includes alkyl groups, which can further include oxygen, nitrogen, sulfur or phosphorous atoms replacing one or more carbons of the hydrocarbon backbone, e.g., oxygen, nitrogen, sulfur or phosphorous atoms. In preferred embodiments, a straight chain or branched chain alkyl has 30 or fewer carbon atoms in its backbone (e.g., C1-C30 for straight chain, C3-C30 for branched chain), preferably 26 or fewer, and more preferably 20 or fewer, and still more preferably 4 or fewer. Likewise, preferred cycloalkyls have from 3-10 carbon atoms in their ring structure, and more preferably have 3, 4, 5, 6 or 7 carbons in the ring structure.

Moreover, the term alkyl as used throughout the specification and sentences is intended to include both "unsubstituted alkyls" and "substituted alkyls," the latter of which refers to alkyl moieties having substituents replacing a hydrogen on one or more carbons of the hydrocarbon backbone. Such substituents can include, for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate; phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulthydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety. It will be understood by those skilled in the art that the moieties substituted on the hydrocarbon chain can themselves be substituted, if appropriate. Cycloalkyls can be further substituted, e.g., with the substituents described above. An "alkylaryl" moiety is an alkyl substituted with an aryl (e.g., phenylmethyl (benzyl)). The term "alkyl" also includes unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six, and still more preferably from one to four carbon atoms in its backbone structure, which may be straight or branched-chain. Examples of lower alkyl groups include methyl, ethyl, n-propyl, i-propyl, tert-butyl, hexyl, heptyl, octyl and so forth. In preferred embodiment, the term "lower alkyl" includes a straight chain alkyl having 4 or fewer carbon atoms in its backbone, e.g., C1-C4 alkyl.

The terms "alkenyl" and "alkynyl" refer to unsaturated aliphatic groups analogous in length and possible substitution to the alkyls described above, but that contain at least one double or triple bond, respectively. For example, the invention contemplates cyano and propargyl groups.

The term "aryl" as used herein, refers to the radical of aryl groups, including 5- and 6-membered single-ring aromatic groups that may include from zero to four heteroatoms, for example, benzene, pyrrole, furan, thiophene, imidazole, benzoxazole, benzothiazole, triazole, tetrazole, pyrazole, pyridine, pyrazine, pyridazine and pyrimidine, and the like. Aryl groups also include polycyclic fused aromatic groups such as naphthyl, quinolyl, indolyl, and the like. Those aryl groups having heteroatoms in the ring structure may also be referred to as "aryl heterocycles," "heteroaryls" or "heteroaromatics." The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a polycycle (e.g., tetralin).

The language "biological activities" of a compound of the invetion includes all activities elicited by compound of the inventions in a responsive cell. It includes genomic and non-genomic activities elicited by these compounds.

"Biological composition" or "biological sample" refers to a composition containing or derived from cells or biopolymers. Cell-containing compositions include, for example, mammalian blood, red cell concentrates, platelet concentrates, leukocyte concentrates, blood cell proteins, blood plasma, platelet-rich plasma, a plasma concentrate, a precipitate from any fractionation of the plasma, a supernatant from any fractionation of the plasma, blood plasma protein fractions, purified or partially purified blood proteins or other components, serum, semen, mammalian colostrum, milk, saliva, placental extracts, a cryoprecipitate, a cryosupernatant, a cell lysate, mammalian cell culture or culture medium, products of fermentation, ascites fluid, proteins induced in blood cells, and products produced in cell culture by normal or transformed cells (e.g., via recombinant DNA or monoclonal antibody technology). Biological compositions can be cell-free. In a preferred form, a suitable biological composition or biological sample is a red blood cell suspension. In some forms, the blood cell suspension includes mammalian blood cells. Preferably, the blood cells are obtained from a human, a non-human primate, a dog, a cat, a horse, a cow, a goat, a sheep or a pig. In preferred forms, the blood cell suspension includes red blood cells and/or platelets and/or leukocytes and/or bone marrow cells.

The term "chiral" refers to molecules which have the property of non-superimposability of the mirror image partner, while the term "achiral" refers to molecules which are superimposable on their mirror image partner.

The term "diastereomers" refers to stereoisomers with two or more centers of dissymmetry and whose molecules are not mirror images of one another.

The term "effective amount" includes an amount effective, at dosages and for periods of time necessary, to achieve the desired result, e.g., sufficient to treat an FLT3 disorder or FLT3 related disorder. An effective amount of compound of the invention may vary according to factors such as the disease state, age, and weight of the subject, and the ability of the compound of the invention to elicit a desired response in the subject. Dosage regimens may be adjusted to provide the optimum therapeutic response. An effective amount is also one in which any toxic or detrimental effects (e.g., side effects) of the compound of the invention are outweighed by the therapeutically beneficial effects.

As used herein, FLT3 disorder or an FLT3 related disorder refers to any disorder or condition that is related to, caused either directly or indirectly by FLT3. FLT3 disorders and related disorders include, for example, immune related disorders and neurodegenerative disorders. Immune related disorders, include, for example, immune related disorder is selected from one or more of organ rejection, bone marrow transplant rejection, non-myeloablative bone marrow transplant rejection, ankylosing spondylitis, arthritis, aplastic anemia, Behcet's disease, type 1 diabetes mellitus, graft-versus-host disease, Graves' disease, autoimmune hemolytic anemia, Wegener's granulomatosis, hyper IgE syndrome, idiopathic thrombocytopenia purpura, rheumatoid arthritis, Crohn's disease, multiple sclerosis, Myasthenia gravis, psoriasis, and lupus, among other autoimmune diseases. It might also be used to enhance bone marrow engraftment after non-myeloablative conditioning regimens, and combinations thereof. Neurodegenerative disorders include, for example, axonal degeneration disorders, including, for example, multiple sclerosis; demyelinating core disorders, such as multiple sclerosis, acute transverse myelitis; and disorders of the motor unit such as amyotrophic lateral sclerosis, infantile spinal muscular atrophy and juvenile spinal muscular atrophy); Creutzfeldt-Jakob disease; or Subacute sclerosing panencephalitis.

A therapeutically effective amount of compound of the invention (i.e., an effective dosage) may range from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a compound of the invention can include a single treatment or, preferably, can include a series of treatments. In one example, a subject is treated with a compound of the invention in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of a compound of the invention used for treatment may increase or decrease over the course of a particular treatment. Treatment can be initiated with smaller dosages, which are less than the optimum dose of the compound. Thereafter, the dosage may be increased by small increments until the optimum effect under the circumstances is reached. For convenience, the total daily dosage may be divided and administered in portions during the day if desired. A therapeutically effective amount and a prophylactically effective anti-viral amount of a compound of the invention of the invention is expected to vary from about 0.1 milligram per kilogram of body weight per day (mg/kg/day) to about 100 mg/kg/day.

As used herein, "obtaining a biological sample from a subject," includes obtaining a sample for use in the methods described herein. A biological sample is described above.

As used herein, identifying a subject in need thereof may be done by self identification or by diagnosis of a healthcare professional.

As used herein, a subject in need thereof, is a subject in need of prophylactic treatment of an FLT3 related disorder or has been diagnosed with an FLT3 related disorder.

The term "enantiomers" refers to two stereoisomers of a compound which are non-superimposable mirror images of one another. An equimolar mixture of two enantiomers is called a "racemic mixture" or a "racemate."

The term "haloalkyl" is intended to include alkyl groups as defined above that are mono-, di- or polysubstituted by halogen, e.g., fluoromethyl and trifluoromethyl.

The term "halogen" designates -F, -Cl, -Br or -I.

The term "hydroxyl" means -OH.

The term "heteroatom" as used herein means an atom of any element other than carbon or hydrogen. Preferred heteroatoms are nitrogen, oxygen, sulfur and phosphorus.

The term "homeostasis" is art-recognized to mean maintenance of static, or constant, conditions in an internal environment.

The language "improved biological properties" refers to any activity inherent in a compound of the invention that enhances its effectiveness in vivo. In a preferred embodiment, this term refers to any qualitative or quantitative improved therapeutic property of a compound of the invention, such as reduced toxicity, e.g" reduced hypercalcemic activity.

The term "optionally substituted" is intended to encompass groups that are unsubstituted or are substituted by other than hydrogen at one or more available positions, typically 1, 2, 3, 4 or 5 positions, by one or more suitable groups (which may be the same or different). Such optional substituents include, for example, hydroxy, halogen, cyano, nitro, C1-C8alkyl, C2-C8 alkenyl, C2-C8alkynyl, C1-C8alkoxy, C2-C8alkyl ether, C3-C8alkanone, C1-C8alkylthio, amino, mono- or di-(C1-C8alkyl)amino, haloC1-C8alkyl, haloC1-C8alkoxy, C1-C8alkanoyl, C2-C8alkanoyloxy, C1-C8alkoxycarbonyl, -COOH, -CONH2, mono- or di-(C1-C8alkyl)aminocarbonyl, -SO2NH2, and/or mono or di(C1-C8alkyl)sulfonamido, as well as carbocyclic and heterocyclic groups. Optional substitution is also indicated by the phrase "substituted with from 0 to X substituents," where X is the maximum number of possible substituents. Certain optionally substituted groups are substituted with from 0 to 2, 3 or 4 independently selected substituents (i.e., are unsubstituted or substituted with up to the recited maximum number of substitutents).

The term "heterocycloalkyl" refers to a nonaromatic, completely saturated 3-9 membered monocyclic, 8-12 membered bicyclic, or 11-14 membered tricyclic ring system comprising 1-3 heteroatoms if monocyclic, 1-6 heteroatoms if bicyclic, or 1-9 heteroatoms if tricyclic, said heteroatoms selected from O, N, S, B, P or Si,. Heterocycloalkyl groups may be optionally substituted with one or more substituents. In one embodiment, 0, 1, 2, 3, or 4 atoms of each ring of a heterocycloalkyl group may be substituted by a substituent. Representative heterocycloalkyl groups include piperidinyl, piperazinyl, 2-oxopiperazinyl, 2-oxopiperidinyl, 2-oxopyrrolidinyl, 4-piperidonyl, tetrahydropyranyl, tetrahydrothiopyranyl, tetrahydrothiopyranyl sulfone, morpholinyl, thiomorpholinyl, thiomorpholinyl sulfoxide, thiomorpholinyl sulfone, 1,3-dioxolane, tetrahydrofuranyl, tetrahydrothienyl, thiirene.

The term "isomers" or "stereoisomers" refers to compounds which have identical chemical constitution, but differ with regard to the arrangement of the atoms or groups in space. The compounds herein are intended to include all isomers and stereoisomers.

The term "modulate" refers to increases or decreases in the activity of a cell in response to exposure to a compound of the invention, e.g., the inhibition of proliferation and/or induction of differentiation of at least a sub-population of cells in an animal such that a desired end result is achieved, e.g., a therapeutic result. In preferred embodiments, this phrase is intended to include viral infections of cells.

The term "obtaining" as in "obtaining the FLT3 inhibitor" is intended to include purchasing, synthesizing or otherwise acquiring the inhibitor.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The term "prodrug" includes compounds with moieties that can be metabolized in vivo. Generally, the prodrugs are metabolized in vivo by esterases or by other mechanisms to active drugs. Examples of prodrugs and their uses are well known in the art (See, e.g., Berge et al. (1977) "Pharmaceutical Salts", J. Pharm. Sci. 66:1-19). The prodrugs can be prepared in situ during the final isolation and purification of the compounds, or by separately reacting the purified compound in its free acid form or hydroxyl with a suitable esterifying agent. Hydroxyl groups can be converted into esters via treatment with a carboxylic acid. Examples of prodrug moieties include substituted and unsubstituted, branch or unbranched lower alkyl ester moieties, (e.g., propionoic acid esters), lower alkenyl esters, di-lower alkyl-amino lower-alkyl esters (e.g., dimethylaminoethyl ester), acylamino lower alkyl esters (e.g., acetyloxymethyl ester), acyloxy lower alkyl esters (e.g., pivaloyloxymethyl ester), aryl esters (phenyl ester), aryl-lower alkyl esters (e.g., benzyl ester), substituted (e.g., with methyl, halo, or methoxy substituents) aryl and aryl-lower alkyl esters, amides, lower-alkyl amides, di-lower alkyl amides, and hydroxy amides. Preferred prodrug moieties are propionoic acid esters and acyl esters. Prodrugs which are converted to active forms through other mechanisms in vivo are also included.

The language "a prophylactically effective amount" of a compound refers to an amount of a compound of the invention, including those of the formula (I) or otherwise described herein which is effective, upon single or multiple dose administration to the patient, in preventing or treating an FLT3 related disorder.

The language "reduced toxicity" is intended to include a reduction in any undesired side effect elicited by a compound of the invention when administered in vivo, e.g., a reduction in the hypercalcemic activity.

The term "sulfhydryl" or "thiol" means -SH.

The term "subject" includes organisms which are capable of suffering from an FLT3 disorder or FLT3 related disorder or who could otherwise benefit from the administration of a compound of the invention of the invention, such as human and non-human animals. Preferred human animals include human patients suffering from or prone to suffering from an FLT3 disorder, FLT3 related disorder or associated state, as described herein. The term "non-human animals" of the invention includes all vertebrates, e.g., mammals, e.g., rodents, e.g., mice, and non-mammals, such as non-human primates, e.g., sheep, dog, cow, chickens, amphibians, reptiles, etc. Susceptible to an FLT3 disorder or FLT3 related disorder is meant to include subjects at risk of developing an FLT3 disorder or FLT3 related disorder, i.e., subjects suffering from immune suppression.

As used herein the term "substituent" or "substituted" means that a hydrogen radical on a compound or group (such as, for example, alkyl, alkenyl, alkynyl, alkylene, aryl, aralkyl, heteroaryl, heteroaralkyl, cycloalkyl, cyclyl, heterocycloalkyl, or heterocyclyl group) is replaced with any desired group that do not substantially adversely affect the stability of the compound. In one embodiment, desired substituents are those which do not adversely affect the activity of a compound. A substituent that substantially affects the activity of a compound is one that causes the IC50 of the compound to be greater than 100 □M. In preferred embodiments, a compound of the invention has an IC50 in an assay or test indicative of activity useful for treatment of IL-12-related (or IL-23- or IL-27-related) diseases or conditions. Such assays are known to one of ordinary skill in the art, and include, e.g., the assays described herein, e.g., the assays of Examples 12-14. In preferred embodiments, the assay is an assay of Example 12 and the compound has an IC50 less than 1.0 mM, more preferably less than 100 □M, more preferably less than 10 □M, more preferably less than 1 □M, more preferably less than 100 nM, and more preferably less than 10 nM. The term "substituted" refers to one or more substituents (which may be the same or different), each replacing a hydrogen atom. Examples of substituents include, but are not limited to, halogen (F, Cl, Br, or I), hydroxyl, amino, alkylamino, arylamino, dialkylamino, diarylamino, cyano, nitro, mercapto, oxo (i.e., carbonyl), thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted,with alkyl, aryl, heteroaryl, halogen, hydroxyl, amino, mercapto, cyano, nitro, oxo (=O), thioxo (=S), or imino (=NRc).

In other embodiments, substituents on any group (such as, for example, alkyl, alkenyl, alkynyl, aryl, aralkyl, heteroaryl, heteroaralkyl, cycloalkyl, cyclyl, heterocycloalkyl, and heterocyclyl) can be at any atom of that group, wherein any group that can be substituted (such as, for example, alkyl, alkenyl, alkynyl, aryl, aralkyl, heteroaryl, heteroaralkyl, cycloalkyl, cyclyl, heterocycloalkyl, and heterocyclyl) can be optionally substituted with one or more substituents (which may be the same or different), each replacing a hydrogen atom. Examples of suitable substituents include, but not limited to alkyl, alkenyl, alkynyl, cyclyl, cycloalkyl, heterocyclyl, heterocycloalkyl, aralkyl, heteroaralkyl, aryl, heteroaryl, halogen, haloalkyl, cyano, nitro, alkoxy, aryloxy, hydroxyl, hydroxylalkyl, oxo (i.e., carbonyl), carboxyl, formyl, alkylcarbonyl, alkylcarbonylalkyl, alkoxycarbonyl, alkylcarbonyloxy, aryloxycarbonyl, heteroaryloxy, heteroaryloxycarbonyl, thio, mercapto, mercaptoalkyl, arylsulfonyl, amino, aminoalkyl, dialkylamino, alkylcarbonylamino, alkylaminocarbonyl, or alkoxycarbonylamino; alkylamino, arylamino, diarylamino, alkylcarbonyl, or arylamino-substituted aryl; arylalkylamino, aralkylaminocarbonyl, amido, alkylaminosulfonyl, arylaminosulfonyl, dialkylaminosulfonyl, alkylsulfonylamino, arylsulfonylamino, imino, carbamido, carbamyl, thioureido, thiocyanato, sulfoamido, sulfonylalkyl, sulfonylaryl, or mercaptoalkoxy.

The phrases "systemic administration," "administered systemically", "peripheral administration" and "administered peripherally" as used herein mean the administration of a compound of the invention(s), drug or other material, such that it enters the patient's system and, thus, is subject to metabolism and other like processes, for example, subcutaneous administration.

With respect to the nomenclature of a chiral center, terms "d" and "1" configuration are as defined by the IUPAC Recommendations. As to the use of the terms, diastereomer, racemate, epimer and enantiomer will be used in their normal context to describe the stereochemistry of preparations. Naturally occurring or synthetic isomers can be separated in several ways known in the art. Methods for separating a racemic mixture of two enantiomers include chromatography using a chiral stationary phase (see, e.g., "Chiral Liquid Chromatography," W.J. Lough, Ed. Chapman and Hall, New York (1989)). Enantiomers can also be separated by classical resolution techniques. For example, formation of diastereomeric salts and fractional crystallization can be used to separate enantiomers. For the separation of enantiomers of carboxylic acids, the diastereomeric salts can be formed by addition of enantiomerically pure chiral bases such as brucine, quinine, ephedrine, strychnine, and the like. Alternatively, diastereomeric esters can be formed with enantiomerically pure chiral alcohols such as menthol, followed by separation of the diastereomeric esters and hydrolysis to yield the free, enantiomerically enriched carboxylic acid. For separation of the optical isomers of amino compounds, addition of chiral carboxylic or sulfonic acids, such as camphorsulfonic acid, tartaric acid, mandelic acid, or lactic acid can result in formation of the diastereomeric salts.

As used herein, "suppressing an immune response" refers to reducing the ability of a cell or an animal, particularly a mammal to mount or sustain an immune response, downregulate the immunostimulatory capacity of a cell, and the like. For example, reducing FLT3 activity is one example of suppressing an immune response, or reducing FTL3 dependent signal transduction. "Suppressing" signaling through FLT3 includes any or all of the following states: slowing, delaying, and as well as stopping. Suppressing also encompasses DCs, NK, and B cells going through apoptosis.

A "therapeutically effective amount" is an amount sufficient to effect beneficial or desired results, including clinical results. An effective amount can be administered in one or more administrations. For purposes of this invention, an effective amount of a therapeutic agent is an amount that is sufficient to palliate, ameliorate, stabilize, reverse, slow or delay the progression of the disease state. It is also an amount of an agent which is effective, upon single or multiple dose administration to the patient or in prolonging the survivability of the patient with an FLT3 disorder or FLT3 related disorder beyond that expected in the absence of such treatment.

As used herein, "treatment" or "treating" a subject includes the application or administration of a therapeutic agent (e.g., a small molecule, an antibody, and an antisense nucleic acid) to a subject, or application or administration of a therapeutic agent to a cell or tissue from a subject, who has a diseases or disorder (e.g., immune related disorder), has a symptom of a disease or disorder, or is at risk of (or susceptible to) a disease or disorder, with the purpose of curing, healing, alleviating, relieving, altering, remedying, ameliorating, improving, or affecting the disease or disorder, the symptom of the disease or disorder, or the risk of (or susceptibility to) the disease or disorder.

As stated above, FLT3 inhibitors include small molecules, anti-sense oligonucleotides, anti-FLT3 antibodies, antigen-binding fragments of anti-FLT3 antibodies, polypeptides, peptidomimetics, nucleic acids encoding peptides, organic molecules, and any combination thereof.

Examples of small molecules useful for FLT3 inhibition include, CEP701, AG 1296, AG 1295, CEP-5214, CEP-7055, PKC412, SU11248, SU5416, SU5614, MLN518, BAY43-9006, CHIR-258, amino-benzimidazole-quinolones, Ki23819, staurosporine derivatives and others which have been or will be discovered. CEP701, AG 1296, AG 1295, (De Angelo, et al., (2003) Phase I clinical results with MLN518, a novel FLT3 antagonist: tolerability, pharmacokinetics and pharmacodynamics [abstract] Blood 102, 219a; Estey, et al., (2003) A Randomized Phase II Trial of the Tyrosine Kinase Inhibitor PKC412 in Patients (pts) with Acute Myeloid Leukemia (AML)/High-Risk Myelodysplastic Syndromes (MDS) Characterized by Wild-Type (WT) or Mutated FLT3 [abstract] Blood 102, 2270a; Heinrich, et al., (2003) MLN518, a potent FLT3 inhibitor, displays synergistic effects with cytarabine and daunorubicin on FLT3 ITD leukemia cell lines [abstract] Blood 102, 330a; Kelly, L. M., et al. (2002c), CT53518, a novel selective FLT3 antagonist for the treatment of acute myelogenous leukemia (AML) Cancer Cell 1, 421-432; Levis, M., et al.,(2002) A FLT3-targeted tyrosine kinase inhibitor is cytotoxic to leukemia cells in vitro and in vivo, Blood 99, 3885-3891; Levis, M., and Small, D. (2003b) Novel FLT3 tyrosine kinase inhibitors. Expert Opin Investig Drugs 12, 1951-1962; O'Farrell, et al., (2003a) SU11248 is a novel FLT3 tyrosine kinase inhibitor with potent activity in vitro and in vivo, Blood 101, 3597-3605; Smith, B. D., et al., (2004) Single agent CEP-701, a novel FLT3 inhibitor, shows biologic and clinical activity in patients with relapsed or refractory acute myeloid leukemia, Blood, in press; Stone, R. M., et al. (2004) PKC 412 FLT3 inhibitor therapy in AML: results of a phase II trial, Ann Hematol 83 Suppl 1, S89-90; Tse, K. F., et al., (2002) Inhibition of the transforming activity of FLT3 internal tandem duplication mutants from AML patients by a tyrosine kinase inhibitor, Leukemia 16, 2027-2036; Tse, K. F., et al., (2001) Inhibition of FLT3-mediated transformation by use of a tyrosine kinase inhibitor, Leukemia 15, 1001-1010; Weisberg, E., et al., (2002) Inhibition of mutant FLT3 receptors in leukemia cells by the small molecule tyrosine kinase inhibitor PKC412, Cancer Cell, 433-443; and Yee, K. W., et al., (2002) SU5416 and SU5614 inhibit kinase activity of wild-type and mutant FLT3 receptor tyrosine kinase, Blood 100, 2941-2949. Preclinical Studies of CHIR258, a Small Molecule Inhibitor that Targets FGFR3, for the Treatment of t(4;14) Multiple Myeloma, Suzanne Trudel et al.; presented at the 95th Annual Meeting of the American Association for Cancer Research, March 2004, in Orlando, Fla.; Durable In Vivo Target Modulation With CHIR258, a Small Molecule Inhibitor of Growth Factor Tyrosine Kinase Receptors, Is Associated With Potent Antitumor Efficacy Using Various Dosing Schedules, Sang Hoon Lee et al.; presented at the 95th Annual Meeting of the American Association for Cancer Research, March 2004, in Orlando, Fla.; In Vivo Anti-Angiogenic and Anti-Tumor Activity Is Associated With Target Modulation by CHIR258, a Small Molecule Inhibitor of Receptor Tyrosine Kinases, Sang Hoon Lee et al.; presented as a Symposium Talk at the Keystone Symposium on Protein Kinases and Cancer: The Promise of Molecular-Based Therapies, February 2004, in Silverthorne, Colo; and Preclinical Pharmacology of FLT-3 Kinase InhibitorCHIR258 in the Treatment of Xenograft Tumors of Human Acute Myelogenous Leukemia, Daniel Menezes et al.; presented at the American Society of Hematology (ASH) Annual Meeting December, 2003, in San Diego, Calif. Ki23819 is disclosed in Leukemia (2005) 19, 930-935, 07 April 2005. .

Further examples of compounds useful in the present methods include compounds described in the following US patents or US patent applications (listed as publication numbers), 20050143442, 20050143382, 20050137241, 20050020570, 20030219827, and 20040106615.

Examples of preferred inhibitors of FLT3 for use in accordance with the invention include indolinone compounds, bis(1H-2-indolyl)-1-methanone compounds, indolocarbazole compounds, and quinoxaline compounds, preferably where such compounds show FLT3 inhibition activity as described herein. Specifically preferred FLT3 inhibitors for use in accordance with the invention include the following: 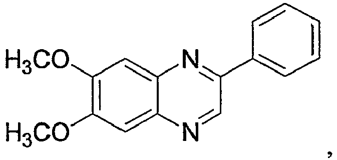 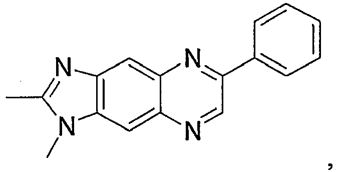 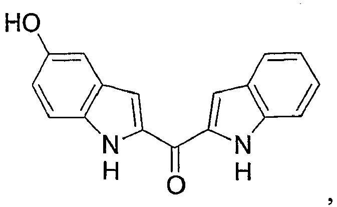 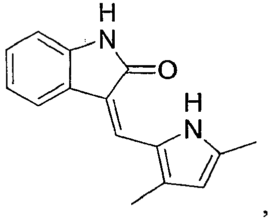 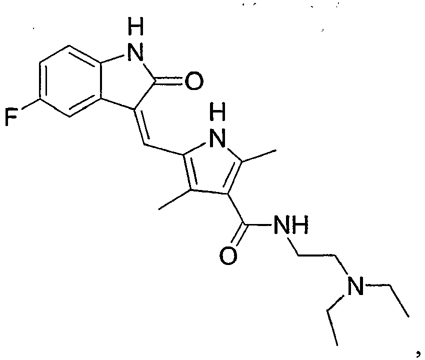 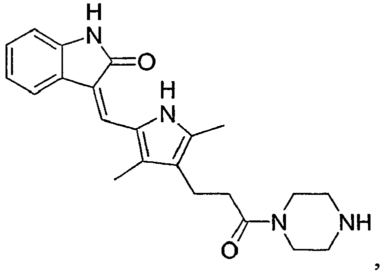 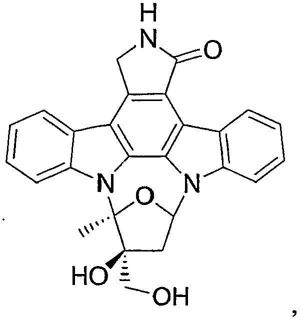 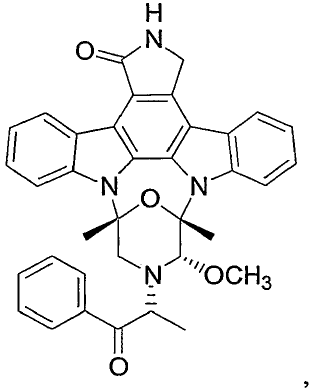 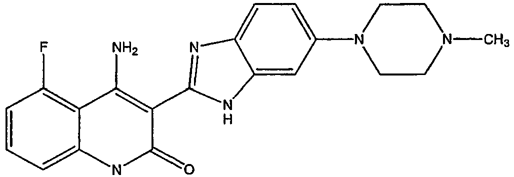 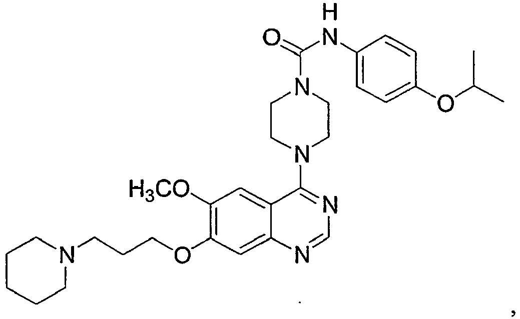 and

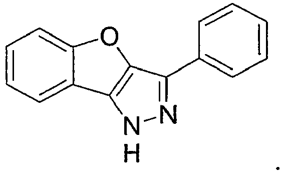

FLT3 inhibitor compounds useful in the invention may also include compounds according to formula I: 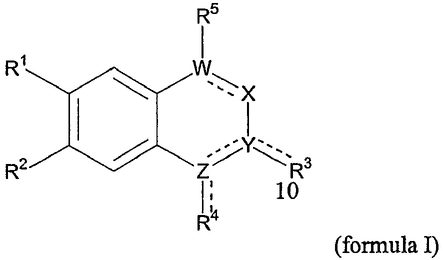 wherein:
W can be C, N, O;
X can be C, N, absent;
Y can be C;
Z can be C, N;
and R groups can be substituted variants of:
   R1 can be H, alkoxy, hydroxyl, heterocyclic alkyl/aryl;
   R2 can be H, F, alkoxy, heterocyclic alkyl/aryl;
   R3 can be H, O, aryl, heteroaryl, C(O)heteroaryl;
   R4 can be H, alkyl, aryl, heteroaryl, CH-heteoaryl, absent;
   R5 can be H, aryl, heteroalkyl/aryl, absent; and
   the dashed lines between WX, WY, YZ, YR3, and ZR4 indicating an optionally double bond between the respective atoms.

The substituents of formula I may be optionally substituted as described herein.

The efficacy of any particular therapeutic agent for the treatment methods can be readily determined. For example, suitable compounds can be identified through the in vitro assays, which assay for the ability to inhibit FLT3 autophosphorylation, which includes the following steps, 1) contacting cells with an potential FLT3 inhibitor, and 2) measuring the phosphorylation state of FLT3 in the cells relative to controls (i.e., same cells not treated with the candidate compound or compounds). Such an autophosphorylation assay that includes those steps 1) and 2) is defined herein as a "standard FLT3 in vitro assay," and is discussed in Tse, K. F., Novelli, E., Civin, C. I., Bohmer, F. D., and Small, D. (2001) Leukemia, 15(7):1001-10, Inhibition of FLT3-mediated transformation by use of a tyrosine kinase inhibitor.

Potentially useful FLT3 inhibitory compounds also can be assessed as disclosed in the examples which follow, which includes: administering a candidate FLT3 inhibitor agent to a mouse having an elevated level of FLT3 receptor activity, and measuring a change in the immune response, wherein a decrease in the immune response indicates that the agent may be useful in treating immune related disorders. The potentially useful compounds are useful without the addition of or co-administration of an interferon antagonist that reduces the activity of type I interferon. However, in certain embodiments, the addition of an interferon antagonist that reduces the activity of type I interferon may be useful. As used herein, an "interferon antagonist" encompasses a antibody, an antigen-binding fragment of an antibody, a polypeptide, a peptidomimetic, a nucleic acid encoding a polypeptide, an organic molecule or any combination thereof which is capable of reducing the activity or function of a type I interferon in a cell within a subject or a cell in vitro.

Preferred therapeutic agents for use in the therapeutic methods of the invention inhibit FLT3 activity by at least 50% and preferably >90% as determined by a standard in vitro FLT3 inhibitor assay. More preferably the agents inhibit FLT3 activity by at least about a 45% or 65%, and still more preferably inhibit FLT3 by at least about 20%, 30%, 40%, 50%, 60%, 70%, 80%, 90% or 100% in a standard in vitro FLT3 inhibition assay.

The term "antibody" or "antibodies" as used herein refers to all types of immunoglobulins, including IgG, IgM, IgA, IgD, and IgE, including Fab or antigen-recognition fragments thereof. The antibodies may be monoclonal or polyclonal and may be of any species of origin, including (for example) mouse, rat, rabbit, horse, or human, or may be chimeric antibodies. See, e.g., M. Walker et al., Molec. Immunol. 26: 403-11 (1989); Morrision et al., Proc. Nat'l. Acad. Sci. 81: 6851 (1984); Neuberger et al., Nature 312: 604 (1984). The antibodies may be recombinant monoclonal antibodies produced according to the methods disclosed in U.S. Pat. No. 4,474,893 (Reading) or U.S. Pat. No. 4,816,567 (Cabilly et al.) The antibodies may also be chemically constructed by specific antibodies made according to the method disclosed in U.S. Pat. No. 4,676,980 (Segel et al.). The antibodies maybe monoclonal, chimeric, anti-idiotypic, humanized, primatized and any combination thereof.

The FLT3 antibodies include antibodies that bind FLT3 in such a way as to inhibit its activation. This includes antibodies, both polyclonal and monoclonal, which bind to FLT3 and inhibit the ability of FL to stimulate it through either blocking FL binding to FLT3 or by inducing conformational changes in FLT3, which block its ability to signal. The term "does not bind" with respect to such antibodies means does not substantially react with as compared to binding to FLT3. Examples of FLT3 antibodies include IMC-NC7 and IMC-EB10 (Li, Y., Li, H., Wang, M., Lu, D., Wu, Y., Bassi, R., Zhang, H., Ludwig, D., Pytowski, B., Kussie, P., Piloto, O., Small, D., Bohlen, P., Witte, L., Zhu, Z., and Hicklin, D.J. Suppression of human leukemia in models that express wild-type or ITD-mutant FLT3 receptor by a fully human anti-FLT3 neutralizing antibody. Blood (Epub 2004, in press, 2004).

The antibodies may include antibodies that bind to phosphorylated FTL3 or unphosphorylated FTL3. The antibodies may be specific for either FTL3 that is phosphorylated or unphosphorylated. Specific, as used herein in the context of antibodies refers to the ability of an antibody to distinguish and to bind exclusively to FTL3.

Antibodies that distinguish phosphorylated or unphosphorylated FLT3 may also be useful for determining the efficacy of the FLT3 inhibitors of the invention or the suppression of the cells or subjects immune response. For example, the level of phosphorylated FLT3 may be determined using such antibodies by, for example, Western blots.

Various procedures known in the art may be used for the production of antibodies to FLT3 or fragment, derivative, homolog or analog of the protein. Antibodies of the invention include, but are not limited to, synthetic antibodies, monoclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), single chain antibodies Fab fragments, F(ab') fragments, disulfide-linked Fvs (sdFv), and anti-idiotypic (anti-Id) antibodies, and epitope-binding fragments of any of the above. In particular, antibodies of the present invention include immunoglobulin molecules and immunologically active portions of immunoglobulin molecules, i.e., molecules that contain an antigen binding site that immunospecifically binds to an antigen (*e.g*., one or more complementarity determining regions (CDRs) of an antibody).

For production of the antibody, various host animals can be immunized by injection with, *e.g*., a native FLT3 protein or a synthetic version, or a derivative of the foregoing. Such host animals include, but are not limited to, rabbits, mice, rats, etc. Various adjuvants can be used to increase the immunological response, depending on the host species, and include, but are not limited to, Freund's (complete and incomplete), mineral gels such as aluminum hydroxide, surface active substances such as lysolecithin, pluronic polyols, polyanions, peptides, oil emulsions, dinitrophenol, and potentially useful human adjuvants such as bacille Calmette-Guerin (BCG) and Corynebacterium parvum. For preparation of monoclonal antibodies directed towards FLT3 or a derivative, fragment, homolog or analog thereof, any technique that provides for the production of antibody molecules by continuous cell lines in culture may be used. Such techniques include, but are not restricted to, the hybridoma technique originally developed by Kohler and Milstein (1975, Nature 256:495-497), the trioma technique (Gustafsson et al., 1991, Hum. Antibodies Hybridomas 2:26-32), the human B-cell hybridoma technique (Kozbor et al., 1983, Immunology Today 4:72), and the EBV hybridoma technique to produce human monoclonal antibodies (Cole et al., 1985, In: Monoclonal Antibodies and Cancer Therapy, Alan R. Liss, Inc., pp. 77-96). In an additional embodiment of the invention, monoclonal antibodies can be produced in germ-free animals utilizing recent technology described in International Patent Application PCT/US90/02545. Antibody fragments that contain the idiotypes of FLT3 can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments. Synthetic antibodies, *e.g*., antibodies produced by chemical synthesis, are useful in the present invention.

Antisense or sense oligonucleotides comprising a single-stranded nucleic acid sequence (either RNA or DNA) capable of binding to a target FLT3 mRNA sequence (forming a duplex) or to the FLT3 sequence in the double-stranded DNA helix (forming a triple helix) can be made according to the invention. Antisense or sense oligonucleotides, according to the present invention, comprise a fragment of the coding region of FLT3 cDNA. Such a fragment generally comprises at least about 14 nucleotides, preferably from about 14 to about 30 nucleotides. The ability to create an antisense or a sense oligonucleotide, based upon a cDNA sequence for a given protein is described in, for example, Stein and Cohen, Cancer Res. 48:2659, 1988 and van der Krol et al., BioTechniques 6:958, 1988. Anti-sense oligonucleotides include, for example, Small, D., Levenstein, M., Kim, E.K., Carow, C., Amin, S., Rockwell, P., Witte, L., Burrow, C., Ratajczak, M., Gewirtz, A.M., and Civin, C.I. STK-1, the human homolog of Flk-2/FLT3, is selectively expressed in CD34+ human bone marrow cells and is involved in the proliferation of early progenitor/stem cells. Proc. Natl. Acad. Sci. 91: 459-463, 1994. The sequence is shown in Figure 9. Useful sequences include the entire sequences shown in Figure 9 or biologically active fragments thereof. One of skill in the art would be able to readily identify useful fragments thereof by routine means.

Binding of antisense or sense oligonucleotides to target nucleic acid sequences results in the formation of complexes that block translation (RNA) or transcription (DNA) by one of several means, including enhanced degradation of the duplexes, premature termination of transcription or translation, or by other means. The antisense oligonucleotides thus may be used to block expression of FLT3 proteins. Antisense or sense oligonucleotides further comprise oligonucleotides having modified sugarphosphodiester backbones (or other sugar linkages, such as those described in WO91/06629) and wherein such sugar linkages are resistant to endogenous nucleases. Such oligonucleotides with resistant sugar linkages are stable in vivo (i.e., capable of resisting enzymatic degradation) but retain sequence specificity to be able to bind to target nucleotide sequences. Other examples of sense or antisense oligonucleotides include those oligonucleotides which are covalently linked to organic moieties, such as those described in WO 90/10443, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oliginucleotide for the target nucleotide sequence.

Antisense or sense oligonucleotides may be introduced into a cell containing the target nucleic acid sequence by any gene transfer method, including, for example, CaPO4-mediated DNA transfection, electroporation, or by using gene transfer vectors such as Epstein-Barr virus. Antisense or sense oligonucleotides are preferably introduced into a cell containing the target nucleic acid sequence by insertion of the antisense or sense oligonucleotide into a suitable retroviral vector, then contacting the cell with the retrovirus vector containing the inserted sequence, either in vivo or ex vivo. Suitable retroviral vectors include, but are not limited to, the murine retrovirus M-MuLV, N2 (a retrovirus derived from M-MuLV), or the double copy vectors designated DCT5A, DCT5B and DCT5C (see PCT Application U.S. No. 90/02656).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs, uracyl, other sugars and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides. A polynucleotide or polynucleotide region has a certain percentage (for example, 80%, 85%, 90%, or 95%) of "sequence identity" to another sequence means that, when aligned, that percentage of bases are the same in comparing the two sequences. This alignment and the percent homology or sequence identity can be determined using software programs known in the art, for example those described in Current Protocols in Molecular Biology (F. M. Ausubel et al., eds., 1987) Supplement 30, section 7.7.18. A preferred alignment program is ALIGN Plus (Scientific and Educational Software, Pennsylvania), preferably using default parameters, which are as follows: mismatch=2; open gap=0; extend gap=2.

In one embodiment of the invention, the interferon antagonist reduces binding of a type I interferon with its receptor. In another embodiment, the interferon antagonist interferes with signal transduction following interferon binding to receptor on cells in the subject. In another embodiment, the interferon antagonist reduces production of interferon by cells in the subject. In another embodiment, the interferon antagonist reduces interferon secretion by cells in the subject. In yet another embodiment, the interferon antagonist reduces bioavailability of interferon in the subject. In a further embodiment, the interferon antagonist is TNF.

As used herein, a "type I interferon" includes IFN-alpha, IFN-beta, IFN-varpi, and IFN-tau Oritani et al. ((2001) Cytokine Growth Factor Rev. 12(4):337-48) provide a description of other examples of a type I interferon.

Interferon antagonists interfere with the interaction between a type I interferon (such as IFN-.alpha.) and its receptor which results in a reduction in the generation of antigen-presenting cells by reducing differentiation of monocytes into DCs which become antigen presenting cells. The reduction in generation of antigen-presenting cells can be achieved by one or more different mechanisms, but the exact mechanism by which this occurs is not crucial to the invention. For example, TNF and/or agonistic anti-TNF receptor antibodies can reduce type I interferon secretion by accelerating the differentiation of pDCs into type I IFN non-producing cells.

There are numerous assays which can be performed to identify whether a compound is an interferon antagonist useful in the present invention. These assays are known to those of skill in the art. One assay is a dendritic cell differentiation assay where a compound to be tested is added to a culture of monocytes under conditions suitable for monocyte differentiation into DCs. The conditions include the addition of either SLE serum or interferon so that the monocytes are induced to differentiate into DCs. Thus, if the compound causes an inhibition of interferon and/or SLE serum driven monocyte differentiation into DCs as compared to differentiation of monocytes in cultures which do not have the compound added, then the compound is an interferon antagonist.

Another assay to identify compounds which are interferon antagonists is a binding assay wherein inhibition of binding of labeled interferon to a receptor on a cell is measured. If a compound is able to inhibit the binding of interferon to its receptor or to cells, having interferon receptors, the compound is an interferon antagonist.

In addition, an assay to determine whether a compound is an interferon antagonist is an assay to measure inhibition of interferon produced by cells in response to triggers that normally induce interferon production and/or secretion, for instance viruses. Thus, if in the presence of the compound and the trigger (e.g., a virus), a cell which would normally produce interferon does not produce interferon, or produces interferon at a reduced level, then the compound is an interferon inhibitor.

In one option an assay to identify a therapeutically effective amount of an interferon antagonist is to determine the amount of interferon antagonist necessary to reduce interferon receptor binding in vitro to serum taken from a subject to be treated. In this example, the concentration necessary to reduce binding by 50% in vitro will be effective therapeutically in vivo.

In one embodiment of the invention, the interferon antagonist comprises an anti-IFN-.alpha. antibody or an antigen-binding fragment thereof. In another embodiment of the invention, the interferon antagonist is TNF. In an embodiment of the invention, the antibody which can be an element of the composition comprises a monoclonal antibody, a chimeric antibody, an anti-idiotypic antibody, a humanized antibody, a primatized antibody and any combination thereof.

The methods of the present invention provide for suppressing, delaying the development of an immune response, treating a heightened immune response, or reducing an immune response of a cell.

Therapeutic methods include selecting or identifying mammalian cells or a mammalian subject suffering from an immune related disorder, particularly as a result of organ transplantation or other immune related disorder. Exemplary cells for treatment include various eukaryotic cells, e.g. DC cells, B-cells, T-cells, and NK cells.

The invention also provides methods of treating an immune related disorder in a subject. The methods comprise administering to the subject a therapeutically effective amount of an FLT3 inhibitor to reduce the activity of FLT3.

Therapeutic methods also provide methods of treating an FLT3 or an FLT3 related disorder in a subject. The subject may be a mammal, wherein the mammal is a human, a primate, a rat, a dog, a cat or a mouse. Methods include, identifying a mammalian subject that that is suffering from or may potentially suffer from, is in a high-risk group for acquiring an immune related disorder, particularly as a result of organ rejection after transplant, bone marrow transplant rejection, non-myeloablative bone marrow transplant rejection, ankylosing spondylitis, arthritis, aplastic anemia, Behcet's disease, type 1 diabetes mellitus, graft-versus-host disease, Graves' disease, hemolytic anemia, Wegener's granulomatosis, hypogammaglobulinemia, hyper IgE syndrome, idiopathic thrombocytopenia purpura, rheumatoid arthritis, Crohn's disease, multiple sclerosis, Myasthenia gravis, psoriasis, and lupus. The methods of the invention may also be used to enhance bone marrow engraftment after non-myeloablative conditioning regimens and any combination thereof. Once identified, the subject is administered a therapeutically effective amount of an FLT3 inhibitor to reduce the activity of FLT3. According to certain forms, the FLT3 inhibitor reduces FLT3 dependent signal transduction in the subject. The methods may further optionally comprise the co-administration of a T-cell inhibitor. Examples of suitable T-cell inhibitors are discussed infra.

According to other forms, the administered FLT3 inhibitor down-regulates the immunostimulatory capacity of a subject. The method for treating a patient's autoimmune disease may comprise the step of administering an amount of FLT3 inhibitor sufficient to decrease the patient's number of dendritic cells. Further included are methods for promoting survival of grafts and transplanted tissues and organs by administering to a patient in need thereof a therapeutically effective amount of an FLT3 inhibitor.

The invention in particular provides methods for treatment, which comprise administration of one or more compounds of the invention to a patient that is undergoing surgery for organ transplantation, lung transplantation, liver transplantation, and the like. Thus, an effective amount of one or more compounds of the present invention could be administered pre-operatively, and/or peri-operatively, and/or postoperatively to reduce immune responses and potential organ rejection.

FTL3 inhibitors may be administered, for example, once a day, twice a day, three times a day, or four times a day. The FTL3 inhibitors may be administered, for example, in tablet form, powered form, liquid for or in capsules. The amount of FTL3 inhibitor administered daily may be increased or decreased based on the weight, age, health, sex or medical condition of the subject.

Methods of the disclosure can be particularly useful in the treatment of mammalian subjects, e.g., humans, to provide immune suppression therapy and/or prophylaxis or provide protection from or treatment for a neurological disorder. Typically, such subjects include those afflicted with immune related disorders such as, graft-versus-host disease, type 1 diabetes mellitus, rheumatoid arthritis, Crohn's disease, multiple sclerosis, Myasthenia gravis, psoriasis, and lupus.

Individuals suitable for treatment by these methods include individuals who have or are suspected of having an immune related disorder, including individuals in the early or late stages of immune related disorders, as well as individuals who have previously been treated or are about to undergo treatment, for example, organ transplantation. Other individuals suitable for the methods described herein are those who are considered high risk for developing an immune related disorder, such as those who have a genetic predisposition to development of arthritis, type 1 diabetes mellitus, rheumatoid arthritis, Crohn's disease, or multiple sclerosis, and/or who have been exposed to an agent(s) which is correlated with development of an immune related disorder.

The presence of an immune related disorder and the suitability of the individual for receiving the methods described herein may be determined by any of the techniques known in the art, including diagnostic methods such as imaging techniques, analysis of serum markers, and biopsy.

In another form, the methods can be administered in conjunction with, i.e., co-administered, other known treatments for immune related disorders, including, but not limited to, protease inhibitors, anti-inflammatory agents, T-cell inhibitors, chemotherapeutic agents, anti-inflammatory agents, anti-pyretic agents radio sensitizing agents, radioprotective agents, urologic agents, anti-emetic agents, and/or anti-diarrheal agents, and the like. Examples include, cyclosporine, mycophenolate mofetil, azathioprine, methotrexate, tacrolimus, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine. Other examples of suitable compounds to be co-administered with the FLT3 inhibitors of the invention may be found in Progress in Drug Research, "Recent advances in immunosuppressants," Bijoy Kundu and Sanjay K. Khare," Vol. 52 (E. Jucker, Ed.), 1999, Birkhäuser Verlag, Basel (Switzerland). The administration of such additional treatments and/or agents are intended to be included in the methods of the present disclosure.

Compounds for use in the methods can be administered intranasally, orally or by injection, e.g., intramuscular, intraperitoneal, subcutaneous or intravenous injection, or by transdermal, intraocular or enteral means. The optimal dose can be determined by conventional means. Compounds for use in the methods are suitably administered to a subject in the protonated and water-soluble form, e.g., as a pharmaceutically acceptable salt of an organic or inorganic acid, e.g., hydrochloride, sulfate, hemi-sulfate, phosphate, nitrate, acetate, oxalate, citrate, maleate, mesylate, etc.

Compounds for use in the methods can be employed, either alone or in combination with one or more other therapeutic agents as discussed above, as a pharmaceutical composition in mixture with conventional excipient, i.e., pharmaceutically acceptable organic or inorganic carrier substances suitable for parenteral, enteral or intranasal application which do not deleteriously react with the active compounds and are not deleterious to the recipient thereof. Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid.monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

For parenteral application, particularly suitable are solutions, preferably oily or aqueous solutions as well as suspensions, emulsions, or implants, including suppositories. Ampules are convenient unit dosages. For enteral application, particularly suitable are tablets, dragees or capsules having talc and/or carbohydrate carrier binder or the like, the carrier preferably being lactose and/or corn starch and/or potato starch. A syrup, elixir or the like can be used wherein a sweetened vehicle is employed. Sustained release compositions can be formulated including those wherein the active component is protected with differentially degradable coatings, e.g., by microencapsulation, multiple coatings, etc. For topical applications, formulations may be prepared in a topical ointment or cream containing one or more compounds of the invention. When formulated as an ointment, one or more compounds of the invention suitably may be employed with either a paraffinic or a water-miscible base. The one or more compounds also may be formulated with an oil-in-water cream base. Other suitable topical formulations include e.g. lozenges and dermal patches. Intravenous or parenteral administration, e.g., sub-cutaneous, intraperitoneal or intramuscular administration are generally preferred.

It will be appreciated that the actual preferred amounts of active compounds used in a given therapy will vary according to the specific compound being utilized, the particular compositions formulated, the mode of application, the particular site of administration, etc. Optimal administration rates for a given protocol of administration can be readily ascertained by those skilled in the art using conventional dosage determination tests conducted with regard to the foregoing guidelines. The desired dose is suitably administered once daily, or several sub-doses, e.g. 2 to 4 sub-doses, are administered at appropriate intervals through the day, or other appropriate schedule.

Also provided is a transgenic non-human animal having a constitutively activated FLT3 receptor. Particularly provided is a transgenic mouse known as the "Tel-FLT3 transgenic mouse," having a constitutively active FLT3 gene inserted into its genome. The Tel-FLT3 transgenic mouse was produced according to standard transgenic technology known in the art. The FLT3 transgenic mice have greatly increased numbers of DCs in their spleens and lymph nodes.

Methods are provided for the use of transgenic animals to test therapeutic agents. For example, method steps include, providing a transgenic animal, preferably a mouse with a constitutively or controllably active FLT3 gene. Methods may also include administering to the animal a therapeutic agent and measuring the animal's immune response to determine the affect of the therapeutic agent. For example, the number of DCs maybe measured, wherein a decrease in the number of DCs indicates a decrease in the immune response of the animal. The number of T cells may also be measured after administration, wherein a decrease in the number of T cells indicates a decrease in the immune response of the animal. The phosphorylation state of FLT3 may also be used as an indication that the immune response of the cell or the subject is suppressed after the administration of the FLT3 inhibitors of the invention. For example, the amount of phosphorylated FLT3 may be decreased in the cell or subject that has been administered an FLT3 inhibitor according to the methods . The transgenic animals according to certain embodiments are useful models for immune related disorders. For example, humans suffering from certain immune related disorders have increased numbers of DCs, T cells, and/or NK cells.

The invention also provides methods for screening a therapeutic agent for activity in vitro and in vivo. For example, a therapeutic agent can be administered to a cell, which is then examined for a decrease in its immune response. Specifically, bone marrow cells, and more specifically, DCs produced from bone marrow of an animal are particularly useful. Other useful cell type and/or lines include, mixed lymphocytes, Tcells and NK cells. For in vitro applications, such as the method of screening therapeutic agents, a multi-well plate or other reaction substrate may be suitably employed.

Methods for screening a therapeutic agent for treating an immune related disorder include, administering the agent to a mouse having an elevated level of FLT3 receptor activity, and measuring a change in the immune response, wherein a decrease in the immune response indicates that the agent may be useful in treating immune related disorders. The change in the immune response is particularly a decreased number of DC cells, NK cells and/or B cells. The change in the immune response may also be measured by determining the level of DC costimulatory protein, wherein a decrease in the level of DC costimulatory protein indicates that the agent may be useful in treating immune related disorders. According to certain forms, the DC costimulatory protein measured is B7-DC. According to certain forms, a measure of the immune response may be a measure of the number of cells compared to other doses of the same therapeutic agent or to a control. The cells measured may be, for example, DC cells, NK cells, spleen cells, mixed bone marrow cells, T cells and mixtures thereof.

According to certain forms, the change in the immune response may be measured by a T cell proliferation assay, wherein a decrease T cell proliferation indicates that the agent may be useful in treating immune related disorders.

The invention also provides methods for screening a therapeutic agent for activity in vivo. For example, a therapeutic agent can be administered to a mammal, which is then examined for a decrease in immune response. Specifically, a transgenic mouse has been generated in which the FLT3 receptor is constitutively activated. The mice have greatly increased numbers of DCs in their spleens and lymph nodes. Knockout mice were generated in which the FLT3 gene has been inactivated. (K. Mackarehtschian, J.D. Hardin, K.A. Moore, et al., Immunity 3, 147-61 (1995)). A decrease in the B lymphocyte progenitor cell numbers was seen, but peripheral B lymphocytes were unaffected. DCs have not been examined in these mice.

Although methods of immune response suppression are exemplified in the discussion below, it is understood that the alternative methods described above are equally applicable and suitable, and that the endpoints of these methods (e.g., efficacy of treatment) are measured using methods standard in the art, including well known diagnostic and assessment methods.

This invention is further illustrated by the following examples, which should not be construed as limiting.

### EXAMPLE 1:

### Treatment of mature DCs with FLT3 inhibitors induces apoptosis in mature DCs.

The survival and function of the DCs was investigated when exposed to FLT3 inhibitors. Mature DCs were incubated in a dose-response analysis to CEP 701, 5214 and AG 1296 in the presence of GM-CSF +/- FL. A decrease in survival and stimulation of the DCs was observed.

Bone marrow (BM) was flushed from the extremities of mice with PBS, red blood cells (RBCs) were lysed in hypotonic buffer, and after washing the cells were plated at 2 x 106 cells/ml in GM-CSF (1000 U/ml) or GM + FL (100 ng/ml) containing medium. On days 2, 4, and 6, non-adherent cells were removed and adherent cells were washed prior to the addition of fresh medium. On day 8, non-adherent mature DCs were harvested, and replated in the absence or presence of CEP-701 at 5 or 50nM. After 24 hours, cells were then stained for FACS analysis. CD11c+ cells were assessed for MHC class II expression and Annexin V binding (Figure 1A). CEP-701 induced apoptosis in DCs generated either in GM-CSF alone or in the combination of GM-CSF and FL. These results have also been confirmed using two other FLT3 inhibitors, including CEP-5214 (data not shown) and AG1296.

Figure 1B shows the results of the stimulation of DCs, generated by flushing BM from the extremities of mice with PBS, lysing the RBCs and washing the cells. After washing, the cells were plated at 2 X 106 cells/ml in GM-CSF (1000 U/ml) + FL (100 ng/ml) containing medium. On days 2, 4, and 6, non-adherent cells were removed and adherent cells were washed prior to the addition of fresh medium. On day 8, non-adherent mature DCs were harvested, and replated in the absence or presence of AG 1296. After 24 hours, cells were stained for FACS analysis. Dendritic cells (CD11c+ cells) were assessed for CD86 expression and Annexin V binding (Figure 1B). AG1296 induced apoptosis in DCs in a dose-dependent fashion. Thus, FLT3 inhibition can induce cell death in DCs in a dose-dependent fashion. As DCs are responsible for presenting antigen to and activating T cells, killing DCs, suppressing DC signaling through FLT3, or preventing DC cell generation by inhibiting FLT3 is likely to have effects on suppressing the T cell arm of the immune system. For DCs to activate T cells, they present antigen bound to MHC molecules and express co-stimulatory proteins on their surfaces.

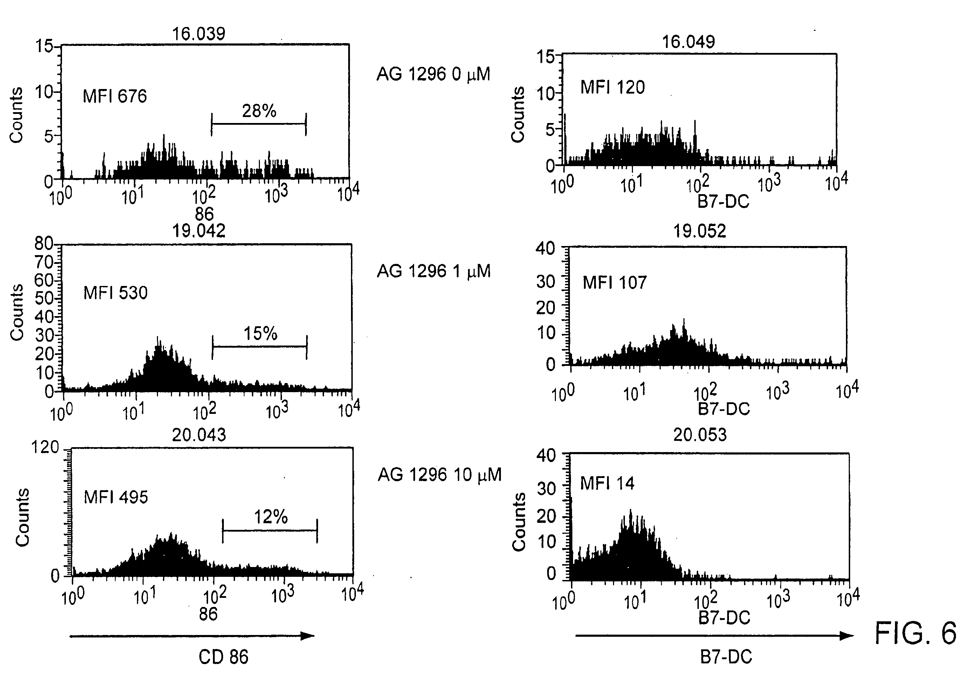

To investigate the role of FL/FLT3 stimulation in regulating costimulatory protein expression by DC, human bone plus IL-4 or GM-CSF plus FL, were analyzed by Northern blotting for expression of B7-DC, a member of the B7 family of co-stimulatory molecules. Figure 1C shows that the addition of FL greatly up-regulates B7-DC expression (GM-CSF alone showed no expression; data not shown). Murine BM cultures showed the same results by FACS. The inhibition of FLT3 signaling led to a down-regulation of B7-DC as well as B7.2 (CD 86) in these DCs (Figure 6). This indicates that a decrease in immune responses may be achieved not only by inducing cell death of DCs, but also by down-regulating their immunostimulatory capacity, thereby enhancing their utility as immunosuppressants.

### EXAMPLE 2:

FLT3 inhibition decreases DC-based proliferation of T cells. For these experiments, DCs were generated as above from BALBc mice. The cells were left untreated or were treated with CEP701 and then plated with C57BL/6 splenocytes at the ratios shown. After 3 days, ³H was added, and the proliferation determined. As Figure 2A shows, CEP701 had a robust effect on decreasing proliferation. In Figure 2b, BALB/c splenocytes were substituted for DCs, in order to test the inhibition of CEP701 on a standard mixed lymphocyte reaction (MLR). As this figure also shows, this response was also inhibited in the presence of CEP701.

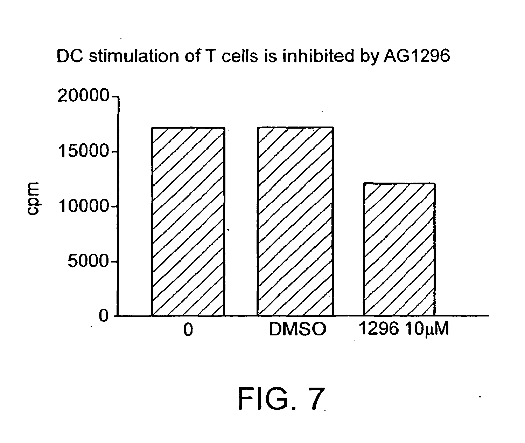

To determined whether T cell stimulatory response would be inhibited as a result of exposure to AG1296, two different T cell proliferative assays were conducted. Figure 2c shows the results of co-incubating spleen cells from allogeneic mice in the presence (10 µm) or absence of inhibitor, In this standard mixed lymphocyte reaction, equal numbers of stimulator and responder cells (10⁶ of each) were mixed in a 96 well plate, incubated for 4 days, pulsed with ³H-thymidine, and proliferation was determined by analyzing incorporation of tritium. DC-stimulated proliferation was also determined by incubating BM derived DCs (day 8, as above) at a ratio of 1:10 with responder allogeneic splenocytes, in the presence (10 µm) or absence of AG1296. As shown in Figure 7, there was a decrease in T cell proliferation induced by DCs in the presence of AG1296.

### EXAMPLE 3:

### Treatment of T cells directly does not inhibit their proliferation.

To show that the inhibition observed in figures 2a and b was due to an effect of the inhibitor on the DCs and not the T cells themselves, 1 x 10⁶ T cells were plated on anti-CD3 coated plates. T cells were then stimulated with anti-CD28 antibody. After 2 days, ³H was added, and the proliferation determined. As the figure shows, no effect on direct stimulation of T cells was observed.

### EXAMPLE 4:

### Treatment of mice with FLT3 inhibitors decreases CD11c populations but not CD3 or B220.

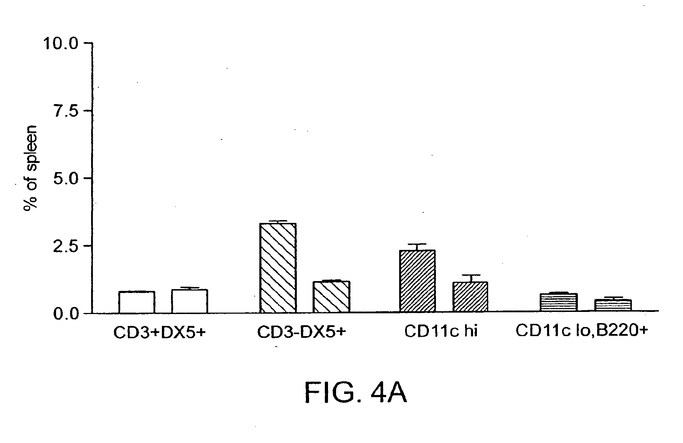

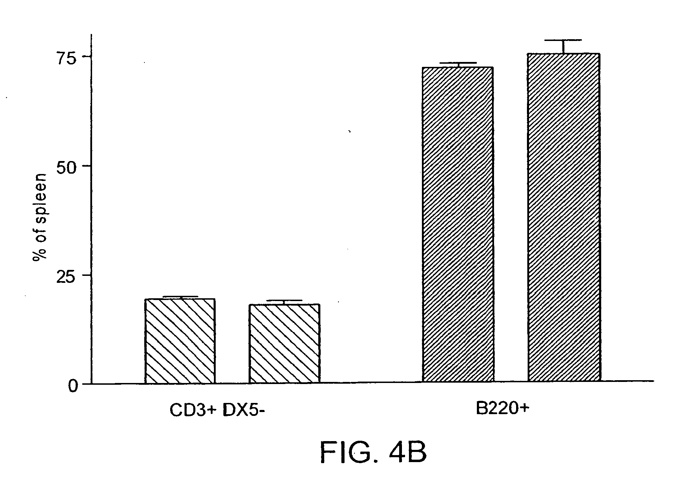

BALB/c mice were treated with 20 mg/kg of CEP701 or vehicle twice a day for 5 days, and then were analyzed for their immune populations. Spleens and lymph nodes were harvested and collagenase digested to release DCs from the stroma. Cells were then washed and analyzed by FACS for the presence of T (CD3+/DX5-), NK (CD3-,DX5+), B (B220+, CD11c-) or DCs (CD11c+). As Figures 4 a and b show, while this treatment led to a decrease in NK and DC populations, no significant change was observed in the B or T cell populations.

### EXAMPLE 5:

### Treatment of mice with FLT3 inhibitors decreases an in vivo immune response, and inhibition of DCs was sufficient to down-regulate an immune response.

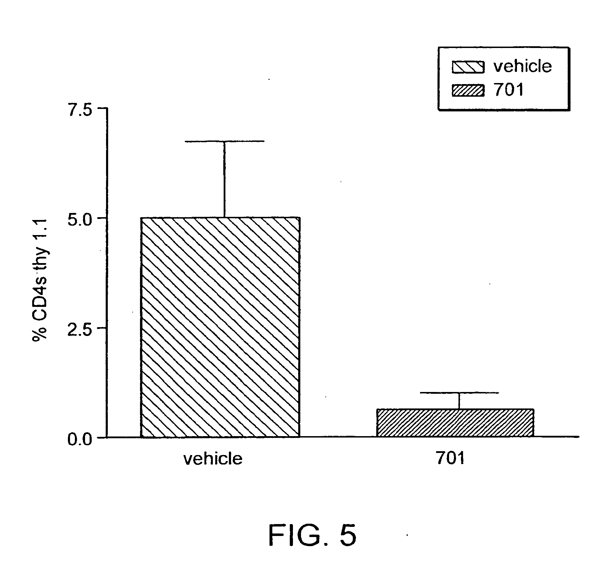

To determine whether FLT3 inhibition would lead to an in vivo down-regulation of an immune response, mice that are transgenic for the influenza hemagluttinin antigen (HA) (the mice are termed "137") were injected with transgenic T cells that are specific for HA (these CD4+ T cells are termed "6.5" and express the congenic T cell marker thy1.1. In this model system, the transferred transgenic T cells expand and induce an autoimmune process. (Huang CT JI 2003). For these experiments, 137 mice were treated with CEP701 as above, for 3 days prior to transfer, and 5 days after transfer. After 5 days, mice were analyzed for expansion of the T cells. As the Figure 5 shows, the CEP701 treatment led to a decrease in the autoreactive T cell expansion, as measured by thy 1.1.

### EXAMPLE 6:

### Monoclonal Antibodies To FLT3

This example illustrates a method for preparing monoclonal antibodies to FLT3. FLT3 is expressed in mammalian host cells such as COS-7 or CV-1/EBNA-1 cells and purified using FLT3:Fc affinity chromatography. Purified FLT3, or a fragment thereof such as the extracellular domain, synthetic peptides or cells that express FLT3 can be used to generate monoclonal antibodies against FLT3 using conventional techniques, for example, those techniques described in U.S. Pat. No. 4,411,993. Briefly, mice are immunized with FLT3 as an immunogen emulsified in an adjuvant, for example, complete Freund's adjuvant, and injected in amounts ranging from 10-100 µg subcutaneously or intraperitoneally. Ten to twelve days later, the immunized animals are boosted with additional FLT3 emulsified in an adjuvant. Mice are periodically boosted thereafter on a weekly to bi-weekly immunization schedule. Serum samples are periodically taken by retro-orbital bleeding or tail-tip excision to test for FLT3 antibodies by dot blot assay or ELISA (Enzyme-Linked Immunosorbent Assay).

Following detection of an appropriate antibody titer, positive animals are provided one last intravenous injection of FLT3 in saline. Three to four days later, the animals are sacrificed, spleen cells harvested, and spleen cells are fused to a murine myeloma cell line, e.g., NS1 or preferably P3 X 63Ag8.653 (ATCC CRL 1580). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against purified FLT3 by adaptations of the techniques disclosed in Engvall et al., Immunochem. 8:871, 1971 and in U.S. Pat. No. 4,703,004. A preferred screening technique is the antibody capture technique described in Beckmann et al., (J. Immunol. 144:4212, 1990) Positive hybridoma cells can be injected intraperitoneally into syngeneic BALB/c mice to produce ascites containing high concentrations of anti-FLT3 monoclonal antibodies. Alternatively, hybridoma cells can be grown in vitro in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to protein A or protein G can also be used, as can affinity chromatography based upon binding to FLT3.

### EXAMPLE 7

### Treatment With FLT3 Inhibitors Ameliorates Established Experimental Autoimmune Encephalomyelitis

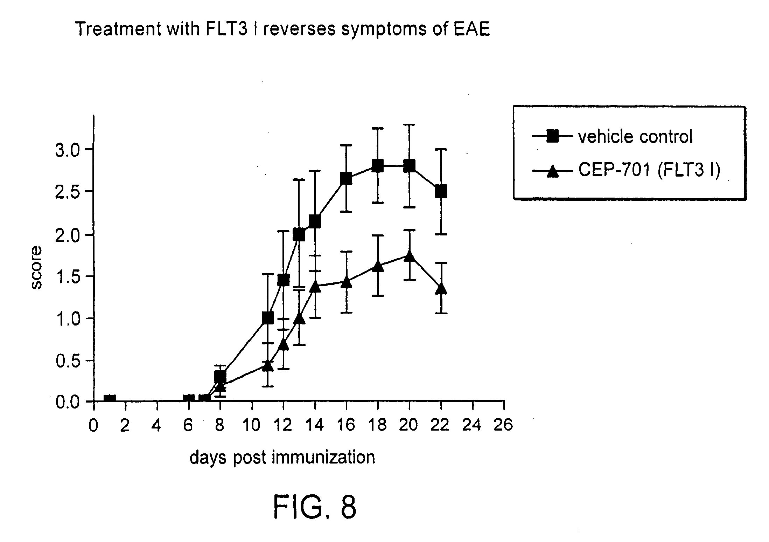

C57BL/6 mice were injected with 100 micrograms of the Established Experimental Allergic Encephalitomyelitis (EAE) inducing peptide MOG 35-55 in CFA with 400 micrograms of Mycobacterium tuberculosis. On the day of immunization and 48 hours later, mice were boosted with pertussis toxin, following standard protocols. Within one day of initial symptoms, mice were injected with either vehicle control or CEP-701 (20 mg/kg), and scored daily by a blinded observer. Scores were as follows: 0 (no symptoms), 0.5 (partial loss of tail tonicity), 1 (full loss of tail tonicity), 2 (affected gait), 2.5 (partial hind limb paralysis) 3 (full hind limb paralysis) 4 (front and hind paralysis) 5 (moribund). 10 mice/ group were treated in the study shown. Figure 8 graphically shows that the mice treated with CEP-701 (FLT3 inhibitor) have lower scores, indicating that the FLT3 inhibitors ameliorated EAE

### EXAMPLE 8

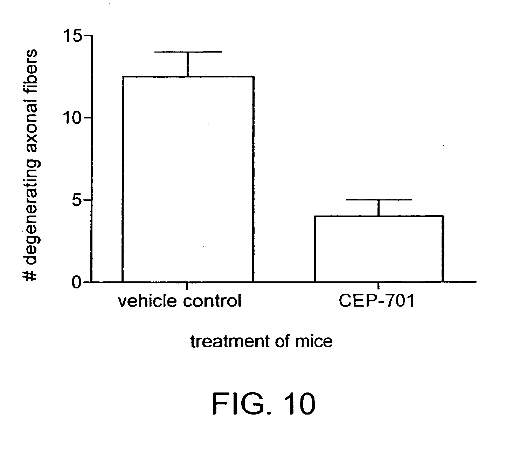

In reference to Figure 10, intact brain and spinal cords were removed from mice that were immunized with MOG peptide, and either treated with the vehicle control or CEP-701 after symptom onset. Following treatment and scoring, the mice were kept for an additional 2 months, then sacrificed and the CNS prepared for evaluation by preservation in formalin. Thin plastic sections (1mm) were generated from cervical level 5, followed by staining in Toluidine blue and axons were visualized. Total axon numbers and actively degenerating fibers in a predefined area of the medial dorsal column were enumerated by an experienced examiner in a blinded fashion. Plots show the number of degenerating axonal fibers in the defined field area for the sections of CEP-701 treated mice compared to vehicle control mice at 210 days post disease induction. These results indicate that inhibiting FLT 3 leads to a decrease in neurodegeneration.

The invention has been described in detail with particular reference to the embodiments thereof. However, it will be appreciated that modifications and improvements within the spirit and teachings of the inventions may be made by those in the art upon considering the present disclosure.

## Claims

1. A FLT3 kinase inhibitor for use in treating a FLT3 disorder or an FLT3 related disorder in a subject,
wherein the FLT3 disorder or FLT3 related disorder is selected from the group, consisting of organ rejection, bone marrow transplant rejection, non-myeloablative bone marrow transplant rejection, ankylosing spondylitis, arthritis, aplastic anemia, Behcet's disease, type 1 diabetes mellitus, graft-versus-host disease, Graves' disease, autoimmune hemolytic anemia, Wegener's granulomatosis, hyper IgE syndrome, idiopathic thrombocytopenia purpura, rheumatoid arthritis, Crohn's disease, Myasthenia gravis, psoriasis, lupus, demyelinating disorders, acute transverse myelitis, Creutzfeldt-Jakob disease, or subacute sclerosing panencephalitis;
and wherein the FLT3 kinase inhibitor is selected from one or more of the group, consisting of
(a) an anti-sense oligo-nucleotide, an anti-FLT3 antibody, an antigen-binding fragment of an anti-FLT3 antibody, a polypeptide, a peptidomimetic, a nucleic acid encoding a peptide,
(b) CEP701, AG 1296, AG 1295, CEP-5214, CEP-7055, PKC412, SU11248, SU5416, SU5614, MLN518, BAY43-9006, CHIR-258, amino-benzimidazole-quinolones, Ki23819, staurosporine,
(c) a compound according to formula 1: 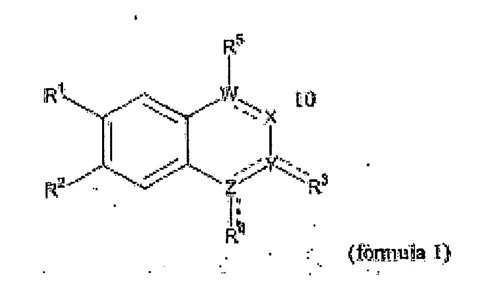 wherein:
W can be C, N, or O;
X can be C, N, absent;
Y can be C;
Z can be C, N;
and R groups can be substituted variants of:
R¹ can be H, alkoxy, hydroxyl, heterocyclic alkyl/aryl;
R² can be H, F, alkoxy, heterocyclic alkyl/aryl;
R³ can be H, O, aryl, heteroaryl, C(O)heteroaryl;
R⁴ can be H, alkyl, aryl, heteroaryl, CH-heteoaryl, absent;
R⁵ can be H, aryl, heteroalkyl/aryl, absent; and
the dashed lines between WX, WY, YZ, YR3, and ZR4 indicating an optionally double bond, wherein the aryl, heteroaryl, heterocyclic alkyl/aryl are optionally substituted, and
(d) a compound of the formula: 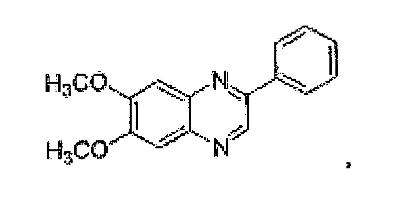 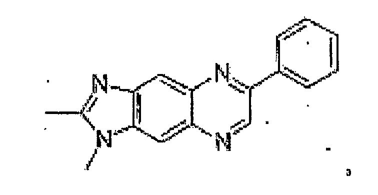 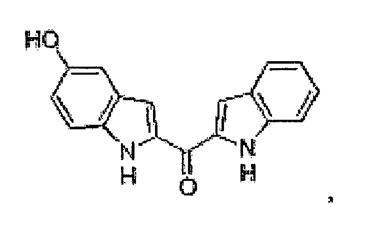 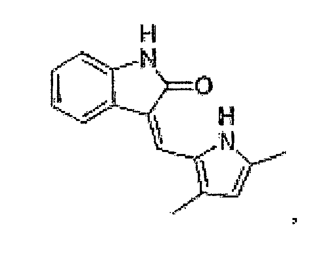 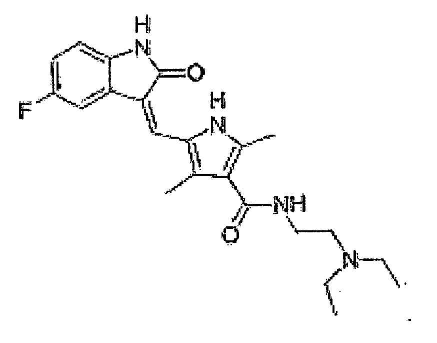 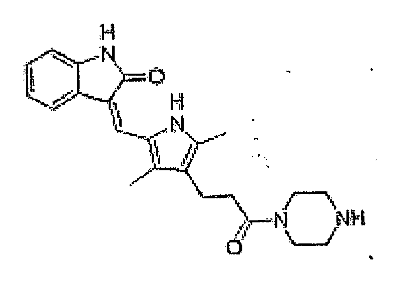 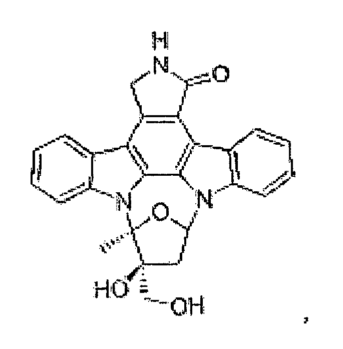 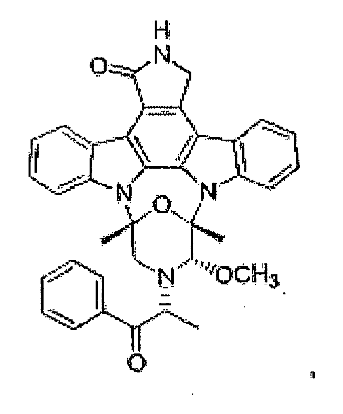 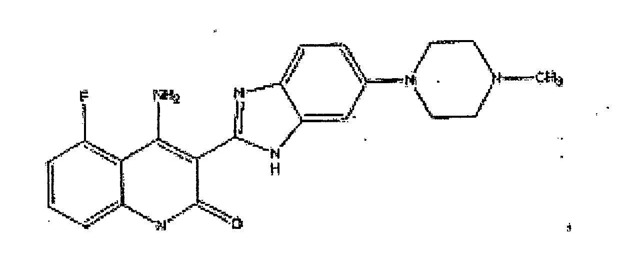 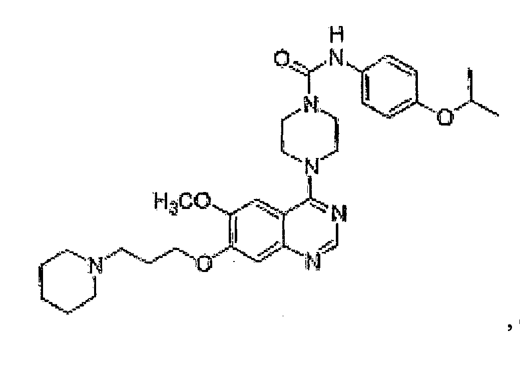 or

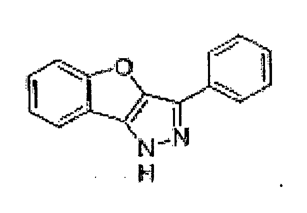

2. The FLT3 kinase inhibitor for use according to claim 1, wherein the anti-FLT3 antibody comprises a monoclonal antibody, a chimeric antibody, a single chain antibody, an anti-idiotypic antibody, a humanized antibody, a fully human antibody, a primatized antibody and any combination thereof.

3. The FLT3 kinase inhibitor for use according to claim 1, wherein the antibody specifically recognizes a phosphorylated form of FLT3.

4. The FLT3 kinase inhibitor for use according to claim 1, wherein the antibody specifically recognizes an unphosphorylated form of FLT3.

5. The FLT3 kinase inhibitor for use according to claim 1, wherein the anti-sense oligonucleotide is selected from one or more of the nucleotide sequence of Figure 9 or a biologically active fragment thereof.

## Patentansprüche

1. FLT3-Kinase-Inhibitor zur Verwendung bei der Behandlung einer FLT3-Störung oder einer FLT3-bedingten Störung bei einem Subjekt,
wobei die FLT3-Störung oder die FLT3-bedingte Störung ausgewählt ist aus der Gruppe, bestehend aus Organabstoßung, Knochenmarktransplantatabstoßung, nicht-myeloablativer Knochenmarktransplantatabstoßung, Spondylarthritis, Arthritis, aplastischer Anämie, Behcet-Krankheit, Diabetes mellitus Typ 1, Graft-versus-host-Krankheit, Graves-Krankheit, autoimmuner hämolytischer Anämie, Wegener-Granulomatose, Hyper-IgE-Syndrom, idiopathischer Thrombocytopenia purpura, rheumatoider Arthritis, Crohnscher Krankheit, Myasthenia gravis, Psoriasis, Lupus, Demyelinations-Krankheiten, akuter transverser Myelitis, Creutzfeldt-Jakob-Krankheit oder subakuter sklerosierender Panencephalitis;
und wobei der FLT3-Kinase-Inhibitor ausgewählt ist aus einem oder mehreren der Gruppe, bestehend aus
(a) einem Antisense-Oligonukleotid, einem Anti-FLT3-Antikörper, einem Antigenbindenden Fragment eines Anti-FLT3-Antikörpers, einem Polypeptid, einem Peptidomimetikum, einer Nukleinsäure, die ein Peptid codiert,
(b) CEP701, AG 1296, AG 1295, CEP-5214, CEP-7055, PKC412, SU11248, SU5416, SU5614, MLN518, BAY43-9006, CHIR-258, Amino-benzimidazol-chinolonen, Ki23819, Staurosporin,
(c) einer Verbindung der Formel 1: 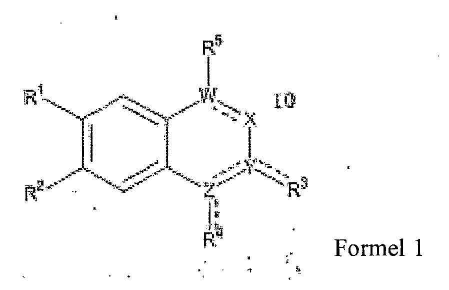 worin
W für C, N oder O stehen kann;
X C, N, nicht vorhanden sein kann;
Y für C stehen kann;
Z für C, N stehen kann;
und R-Gruppen substituierte Varianten sein können von:
R¹ kann H, Alkoxy, Hydroxyl, heterocyclisches Alkyl/Aryl sein;
R² kann H, F, Alkoxy, heterocyclisches Alkyl/Aryl sein;
R³ kann H, O, Aryl, Heteroaryl, C(O)Heteroaryl sein;
R⁴ kann H, Alkyl, Aryl, Heteroaryl, CH-Heteroaryl, nicht vorhanden sein;
R⁵ kann H, Aryl, Heteroalkyl/Aryl, nicht vorhanden sein und
die gestrichelten Linien zwischen WX, WY, YZ, YR3 und ZR4 geben eine optionale Doppelbindung an, wobei das Aryl, Heteroaryl, heterocyclische Alkyl/Aryl gegebenenfalls substituiert ist und
(d) einer Verbindung der Formel : 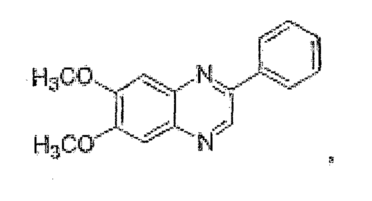 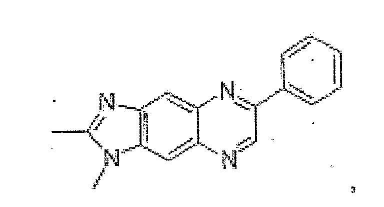 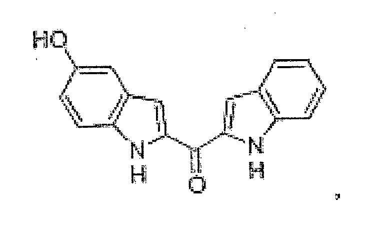 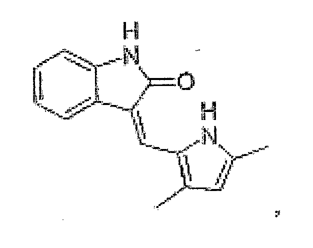 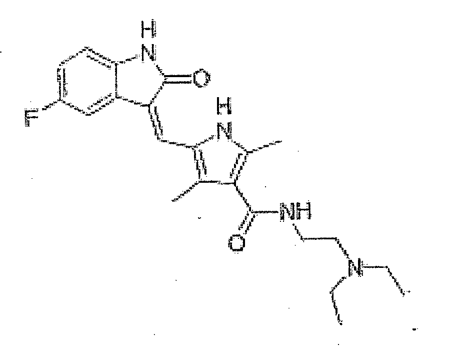 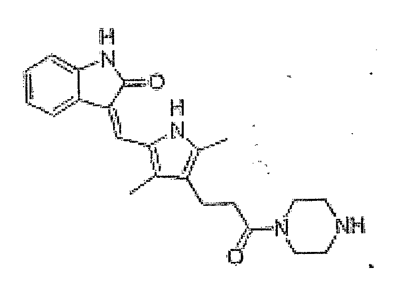 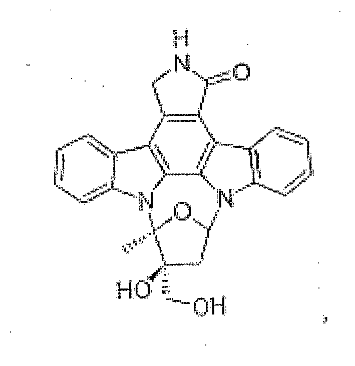 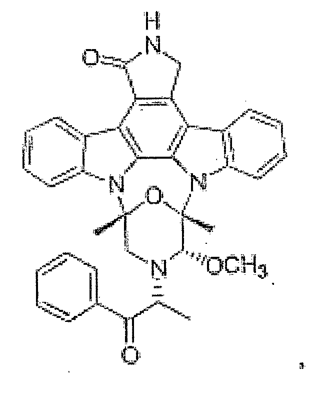 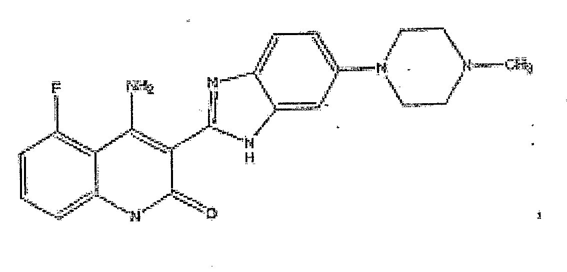 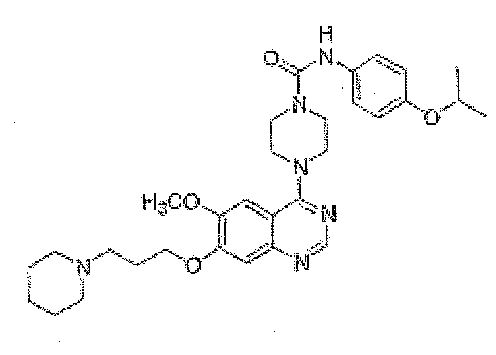 oder

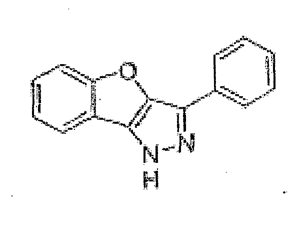

2. FLT3-Kinase-Inhibitor zur Verwendung gemäß Anspruch 1, wobei der Anti-FLT3-Antikörper einen monoklonalen Antikörper, einen chimären Antikörper, einen Einzelkettenantikörper, einen Anti-idiotypischen Antikörper, einen humanisierten Antikörper, einen vollständig humanen Antikörper, einen primatisierten Antikörper und eine beliebige Kombination davon umfasst.

3. FLT3-Kinase-Inhibitor zur Verwendung gemäß Anspruch 1, wobei der Antikörper spezifisch eine phosphorylierte Form von FLT3 erkennt.

4. FLT3-Kinase-Inhibitor zur Verwendung gemäß Anspruch 1, wobei der Antikörper spezifisch eine unphosphorylierte Form von FLT3 erkennt.

5. FLT3-Kinase-Inhibitor zur Verwendung gemäß Anspruch 1, wobei das Antisense-Oligonukleotid aus einer oder mehreren der Nukleotidsequenzen von Figur 9 oder einem biologisch aktiven Fragment davon ausgewählt ist.

## Revendications

1. Inhibiteur de la FLT3 kinase pour une utilisation dans le traitement d'un trouble de la FLT3 ou d'un trouble associé à la FLT3 chez un sujet,
où le trouble de la FLT3 ou le trouble associé à la FLT3 est choisi dans le groupe constitué d'un rejet d'organe, un rejet de transplantation de moelle osseuse, un rejet de transplantation de moelle osseuse non myéloablative, la spondylarthrite ankylosante, l'arthrite, l'anémie aplasique, la maladie de Behçet, le diabète sucré de type 1, la maladie du greffon contre l'hôte, la maladie de Basedow, l'anémie hémolytique auto-immune; la granulomatose de Wegener, le syndrome hyper-IgE, le purpura thrombocytopénique idiopathique, la polyarthrite rhumatoïde, la maladie de Crohn, la myasthénie grave, le psoriasis, le lupus, des troubles démyélinisants, la myélite transverse aiguë, la maladie de Creutzfeldt-Jakob, ou la panencéphalite sclérosante subaiguë ;
et où l'inhibiteur de la FLT3 kinase est choisi parmi un ou plusieurs du groupe constitué de
(a) un oligonucléotide antisens, un anticorps anti-FLT3, un fragment de liaison d'un antigène d'un anticorps anti-FLT3, un polypeptide, un peptidomimétique, un acide nucléique codant pour un peptide,
(b) CEP701, AG 1296, AG 1295, CEP-5214, CEP-7055, PKC412, SU11248, SU5416, SU5614, MLN518, BAY43-9006, CHIR-258, des amino-benzimidazole-quinolones, Ki23819, la staurosporine,
(c) un composé selon la formule 1 : 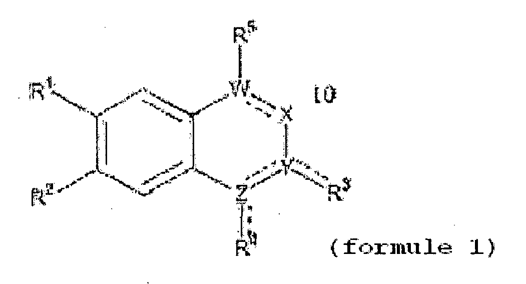 dans laquelle :
W peut représenter C, N, ou O ;
X peut représenter C, N, ou être absent ;
Y peut représenter C ;
Z peut représenter C, N ;
et les groupes R peuvent être des variants substitués de :
R¹ peut représenter H, un groupe alcoxy, hydroxyle, alkyle/aryle hétérocyclique ;
R² peut représenter H, F, un groupe alcoxy, alkyle/aryle hétérocyclique ;
R³ peut représenter H, O, un groupe aryle, hétéroaryle, C(O)hétéroaryle ;
R⁴ peut représenter H, un groupe alkyle, aryle, hétéroaryle, CH-hétéroaryle, ou être absent ;
R⁵ peut représenter H, un groupe aryle, hétéroalkyle/aryle, ou être absent ; et
les lignes en pointillés entre WX, WY, YZ, YR3, et ZR4 indiquent une double liaison éventuelle, où les groupes aryle, hétéroaryle, alkyle/aryle hétérocyclique sont éventuellement substitués, et
(d) un composé de formule : 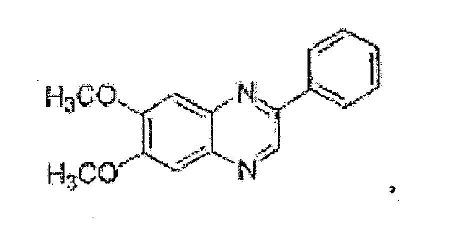 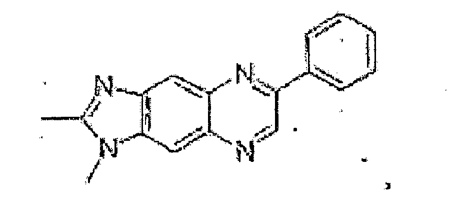 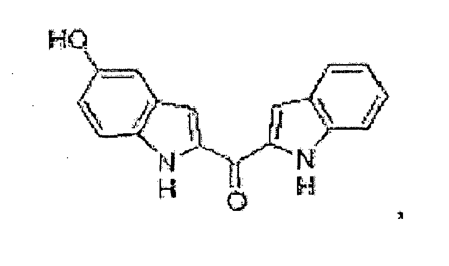 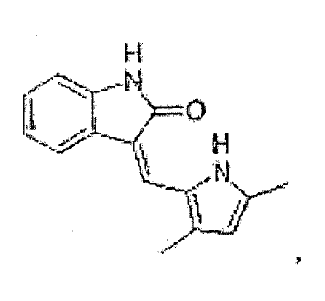 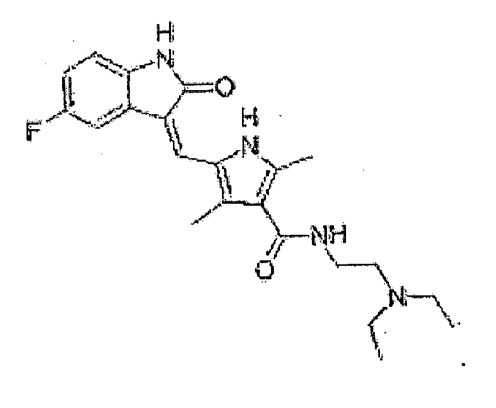 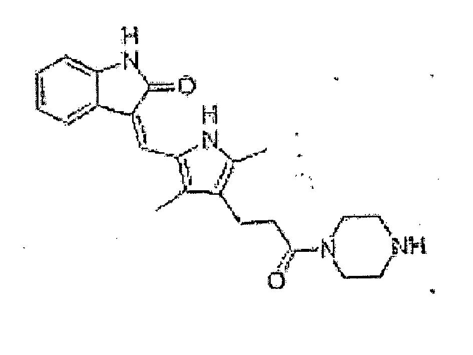 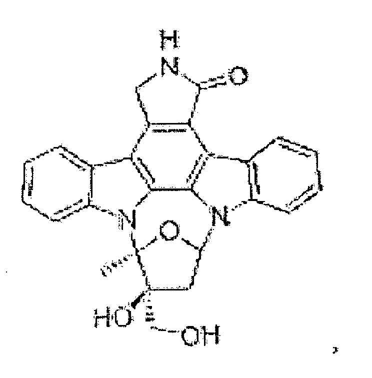 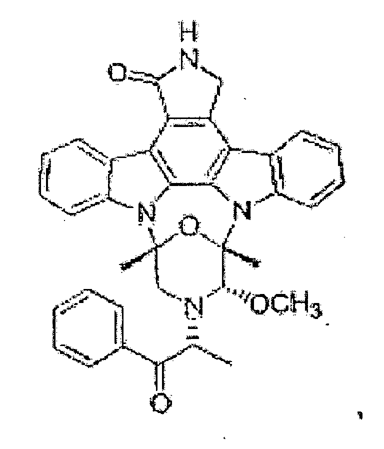 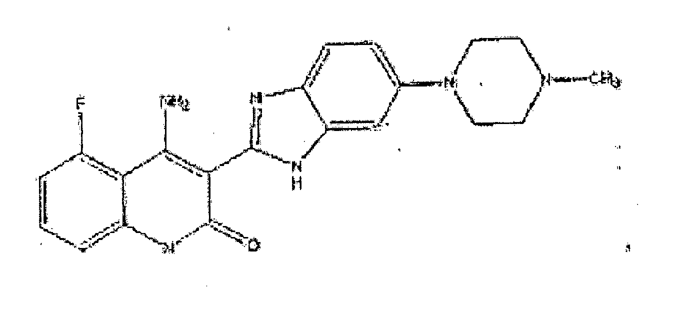 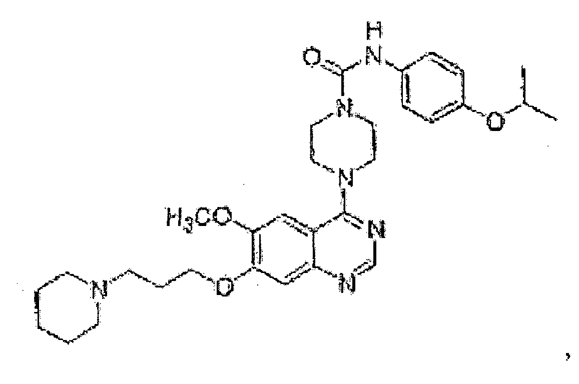 ou

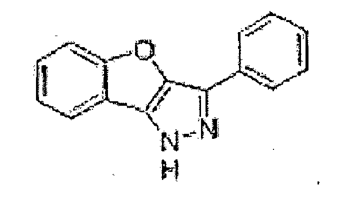

2. Inhibiteur de la FLT3 kinase pour une utilisation selon la revendication 1, où l'anticorps anti-FLT3 comprend un anticorps monoclonal, un anticorps chimérique, un anticorps à chaîne unique, un anticorps anti-idiotypique, un anticorps humanisé, un anticorps totalement humain, un anticorps primatisé et toute combinaison de ceux-ci.

3. Inhibiteur de la FLT3 kinase pour une utilisation selon la revendication 1, où l'anticorps reconnaît spécifiquement une forme phosphorylée de la FLT3.

4. Inhibiteur de la FLT3 kinase pour une utilisation selon la revendication 1, où l'anticorps reconnaît spécifiquement une forme non phosphorylée de la FLT3.

5. Inhibiteur de la FLT3 kinase pour une utilisation selon la revendication 1, où l'oligonucléotide antisens est choisi parmi un ou plusieurs de la séquence nucléotidique de la figure 9 ou d'un fragment biologiquement actif de celle-ci.
